(19) Europäisches Patentamt
European Patent Office
Office européen des brevets

(11) **EP 3 166 579 B1**

(12) **FASCICULE DE BREVET EUROPEEN**

(45) Date de publication et mention
de la délivrance du brevet:
**27.11.2019 Bulletin 2019/48**

(21) Numéro de dépôt: **15732728.9**

(22) Date de dépôt: **01.07.2015**

(51) Int Cl.:
*A61K 8/34* (2006.01)   *A61K 8/60* (2006.01)
*A61Q 13/00* (2006.01)   *A61Q 15/00* (2006.01)
*A61K 8/73* (2006.01)   *A61K 8/11* (2006.01)
*A61K 8/25* (2006.01)   *A61K 8/26* (2006.01)
*A61K 8/29* (2006.01)   *A61K 8/19* (2006.01)

(86) Numéro de dépôt international:
**PCT/EP2015/065007**

(87) Numéro de publication internationale:
**WO 2016/005248 (14.01.2016 Gazette 2016/02)**

(54) **POUDRE ANHYDRE LIBRE OU COMPACTE A BASE DE PARTICULES ENCAPSULANT UN AGENT BENEFIQUE**

WASSERFREIE PUDER IN FREIE ODER KOMPAKT FORM BEZOGEN AUF PARTIKELN ENTHALTEND VERKAPSELTER PFLEGESTOFF

ANHYDROUS POWDER IN FREE OR COMPACT FORM BASED ON PARTICLES ENCAPSULATING A BENEFIT AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.07.2014 FR 1456632**

(43) Date de publication de la demande:
**17.05.2017 Bulletin 2017/20**

(73) Titulaire: **L'Oréal**
**75008 Paris (FR)**

(72) Inventeurs:
• **MALLE, Gérard**
**77580 Villiers S/morin (FR)**
• **LUUKAS, Tiina**
**93270 Sevran (FR)**
• **BARA, Isabelle**
**94210 La Varenne St Hilaire (FR)**

(74) Mandataire: **L'Oreal**
**Service D.I.P.I.**
**9, rue Pierre Dreyfus**
**92110 Clichy (FR)**

(56) Documents cités:
**DE-A1-102008 035 013    JP-A- 2008 156 236**
**US-A1- 2004 029 750**

• **MARTIN ET AL: "Encapsulation and Co-Precipitation Processes with Supercritical Fluids:Applications with Essential Oils", THE OPEN CHEMICAL ENGINEERING JOURNAL,, vol. 4, 1 janvier 2010 (2010-01-01), pages 31-41, XP002737531,**

Remarques:
Le dossier contient des informations techniques présentées postérieurement au dépôt de la demande et ne figurant pas dans le présent fascicule.

**Description**

La présente invention concerne une composition anhydre sous forme de poudre libre ou compacte comprenant:

1) au moins des particules comprenant un cœur contenant au moins un agent bénéfique et une enveloppe entourant le coeur; ladite enveloppe étant constituée d'au moins un polysaccharide modifié hydrophobe choisi parmi les $C_5$-$C_{20}$-alcényl succinate d'amidon et d'au moins un glucide hydrosoluble choisi parmi les maltodextrines ayant un dextrose équivalent allant de 4 à 20; et lesdites particules présentant simultanément une densité de poudre versée allant de 300,0g/l à 600,0g/l et une densité absolue supérieure à 1,0 ; et les dites particules étant sphériques et ayant un diamètre moyen en nombre allant de 1 à 30$\mu$m, et un diamètre moyen en volume allant de 5 à 150$\mu$m; et
2) éventuellement au moins une charge autre que lesdites particules.

L'invention concerne également un procédé cosmétique de soin et/ou d'hygiène et/ou de de conditionnement et/ou de parfumage et/ou de maquillage d'une matière kératinique consistant à appliquer sur ladite matière kératinique une composition telle que définie précédemment.

L'invention concerne en outre un procédé de traitement cosmétique des odeurs corporelles et éventuellement de la transpiration humaine consistant à appliquer sur une matière kératinique une composition telle que définie précédemment comprenant au moins un actif déodorant et/ou un actif anti-transpirant sous forme libre (non encapsulé) et/ou sous forme encapsulée.

De nombreuses formes galéniques cosmétiques permettent de délivrer des agents bénéfiques notamment des produits cosmétiques ou dermatologiques pour la peau, les lèvres, les cheveux, des produits de maquillage; des produits pour le soin des bébés comme les talcs; des produits pharmaceutiques ; des produits à usage vétérinaire notamment des produits d'hygiène et/ou le soin comme les litières pour animaux; des produits d'entretien ménager comme les produits pour le soin et/ou le nettoyage du linge (lessives, adoucissants), les produits de vaisselle, les produits de nettoyage et/ou d'entretien des appareils électroménagers, les produits de nettoyage et/ou d'entretien des sols, carrelages, bois etc ; des produits sanitaires comme des désodorisants, des produits détartrants, des produits déboucheurs de canalisations; des produits issus de l'industrie agro-alimentaire; des produits issus de l'agriculture; des produits phytosanitaires.

Parmi celles-ci, les formes poudre sont utilisées dans de nombreux domaines industriels en particulier la cosmétique. Elles permettent notamment en cosmétique d'apporter de la douceur et de la matité. On connaît notamment des poudres libres ou compactes

Les poudres précitées ont principalement pour fonction d'apporter de la matité et/ou de la couleur, et voire, pour celles plus particulièrement destinées à la peau du visage, d'améliorer la tenue d'une poudre fond de teint ou, si utilisées seules, pour donner de la couvrance. Ces formes galéniques sont particulièrement appréciées par les utilisatrices au regard de leur légèreté, douceur et aspect non collant ou sec au toucher.

Elles sont principalement utilisées dans le domaine du maquillage (fond de teint, fard à paupière,...) mais peuvent également être valorisées dans des produits de soin, d'hygiène ou de protection des matières kératiniques comme la peau ou le cheveu tels que les talcs pour le corps, les shampooings secs, les déodorants en poudre.

D'une manière générale, ces compositions sous forme de poudre comprennent des charges et dans le cadre du maquillage, éventuellement associées à des agents de coloration, la quantité de ces derniers étant modulée pour procurer l'effet de maquillage recherché.

Ces poudres sont généralement appliquées sur la peau à l'aide d'un applicateur comme par exemple une éponge, une houppette ou un pinceau ou bien d'un dispositif du type salière pour les talcs pour le corps ou les shampooings secs pour les cheveux.

Le but de la présente invention est de proposer de nouvelles compositions cosmétiques de type poudre libre ou compacte anhydre comprenant au moins un agent bénéfique encapsulé dans des particules qui seraient étanches à l'abri de l'humidité c'est-à-dire inodores si l'actif est un parfum

-     les dites particules présentant une densité de poudre versée faible pour faciliter leur formulation de façon homogène sans former d'agrégats et obtenir ainsi des poudres très légères

-     lesdites particules devant également être compatibles avec les ingrédients habituels de ces formulations et suffisamment résistantes pour pouvoir être formulées en « poudre» sans être endommagées

-     le dit agent bénéfique contenu dans les microcapsules pouvant être libéré de façon quasi immédiate, progressive et répétable sur la peau, le cheveu et les phanères au contact de l'eau

On sait qu'il existe une nécessité dans de nombreux domaines industriels, de protéger un certain nombre de molécules fragiles ou volatiles et de contrôler leur relargage vers un milieu extérieur.

Un des moyens permettant d'atteindre un tel but est leur encapsulation. Cette encapsulation a pour objectif de diminuer l'évaporation et le transfert vers l'environnement de la matière active, soit au cours du stockage, soit au cours de l'élaboration des produits ou encore au cours de leur utilisation. Elle peut également permettre de rendre le matériau plus facile d'utilisation en le diluant et en favorisant sa répartition homogène au sein du support.

La micro-encapsulation regroupe l'ensemble des technologies permettant l'enrobage ou le piégeage de principes actifs sous forme solide, liquide, ou gazeuse au sein de particules individualisées dont la taille s'échelonne entre quelques microns et quelques millimètres. Si ces microparticules sont creuses (vésiculaires) on parle de microcapsules, si elles sont pleines (matricielles) on parle de microsphères. Leur taille varie de $1\,\mu m$ à plus de $1000\,\mu m$. Ces microparticules peuvent être biodégradables ou non et peuvent contenir entre 5 et 90% (en masse) de substance active.

Les substances actives encapsulées sont d'origines très variées: principes actifs pharmaceutiques, cosmétiques, additifs alimentaires, produits phytosanitaires, essences parfumées, micro-organismes, cellules, ou encore catalyseurs de réaction chimique...

Tout l'intérêt des microparticules d'encapsulation réside dans la présence d'une membrane polymérique, qui isole et protège le contenu du milieu extérieur. Selon les cas, la membrane sera détruite lors de l'utilisation pour libérer son contenu (exemple : encarts publicitaires "scratch and sniff" libérant le parfum lorsqu'on écrase les microcapsules), ou bien la membrane restera présente tout le long de la libération du contenu, dont elle contrôlera la vitesse de diffusion (exemple: encapsulation de médicaments pour libération ralentie).

Les matériaux enrobants sont généralement des polymères d'origine naturelle ou synthétique, hydrophobes ou hydrophiles, ou bien des lipides.

Les principaux procédés pour réaliser l'encapsulation de substances dans des microparticules sont la polymérisation interfaciale, la réticulation interfaciale, l'émulsion suivie d'une évaporation ou d'une extraction du solvant, la double émulsion évaporation/extraction de solvant, le spray-drying, le prilling, la coacervation.

Dans les brevets US 5 508 259, DE 10 2008 035 013 et US 2004/0029750, on a proposé des compositions parfumantes non aqueuses, comprenant des parfums encapsulés dans des capsules solubles dans l'eau. Les capsules décrites dans le brevet US 5 508 259 sont obtenues par des techniques d'encapsulation conventionnelles et en particulier le spray-drying d'une émulsion constituée d'un substrat solide filmogène en combinaison avec un agent émulsionnant et un mélange d'ingrédients de parfumerie. Le substrat solide filmogène est notamment choisi parmi l'acétate de polyvinyle, l'alcool polyvinylique, des dextrines, de l'amidon naturel ou modifié, les gommes végétales, les pectines, les xanthanes, les alginates, carraghénanes ou encore les dérivés de cellulose tels que par exemple la carboxyméthylcellulose, la méthylcellulose ou l'hydroxyéthylcellulose. L'émulsion est ensuite déshydratée par un procédé classique d'atomisation (spray-drying), qui consiste, tel que décrit à l'exemple 1, à la pulvériser en fines gouttelettes dans un atomiseur avec un débit de 50kg/h et une pression de 0,45 bars, au contact d'un courant d'air de $320\,m^3$/h chauffé à 350°C afin d'évaporer l'eau, ce qui permet d'obtenir une poudre fine avec un diamètre de particules compris entre 20 et 80microns et contenant 20% en poids de parfum.

Cependant, on a remarqué que les particules obtenues par ce procédé étaient fortement odorantes à l'état sec à cause de la présence de parfum libre (non encapsulé), qu'elles étaient principalement constituées d'agglomérats pouvant nuire à l'homogénéité du produit et gêner la bonne application du produit et ne possédaient pas les caractéristiques de densité adaptées à l'objectif de l'invention.

Dans le brevet US 6 200 949, on a décrit également un procédé de formation d'une matière particulaire contenant un parfum hydrophile comprenant les étapes successives consistant à former une émulsion aqueuse de parfum contenant 40 à 60% en poids d'eau, 3 à 30% en poids de maltodextrine, 10 à 40% en poids d'amidon modifié hydrophobe, puis à la sécher par pulvérisation dans un atomiseur (courant d'air de $420\,m^3$/h chauffé à 204°C de sorte que les particules sont formées avec une taille moyenne d'environ 3 à environ 10 microns et une teneur en parfum de 15 à 50% en poids. Cependant, les particules obtenues par ce procédé sont fortement odorantes à l'état sec à cause de la présence de parfum libre (non encapsulé), sont principalement constituées d'agglomérats, peuvent nuire à l'homogénéité du produit et ne possèdent pas les caractéristiques de densité adaptées à l'objectif de l'invention.

Il est donc très important de pouvoir disposer de particules d'encapsulation étanches qui ne libèrent leur contenu qu'à la demande (en réponse à l'humidité ambiante, notamment dans les zones climatiques humides, en réponse à la transpiration corporelle, au shampooing ou sous la douche, etc...), d'une part pour garantir la protection dans le temps de l'actif encapsulé surtout s'il est fragile et/ou volatil et d'autre part pour éviter les interactions avec les autres ingrédients de la formule. Lorsque l'agent bénéfique encapsulé est un ingrédient de parfumerie et/ou un parfum complet, il est d'autant plus important que l'encapsulation soit totale, ce qui conduit à des capsules inodores en formules anhydres permettant au formulateur de les associer ou non avec n'importe quel un parfum libre de son choix (identique ou différent) sans risque d'interactions ou de perturbation de la note parfumée choisie.

Dans le brevet EP1917098B1, on a proposé un procédé de préparation de produits d'encapsulation par précipitation, lequel procédé emploie :

- une émulsion pouvant être pompée comprenant (i) une phase continue contenant un solvant et un soluté formant une matrice dissout dans ledit solvant et (ii) une phase dispersée ;

- un extracteur comprenant un gaz supercritique, sous-critique ou liquéfié ;

ledit solvant étant sensiblement plus soluble dans l'extracteur que ledit soluté formant une matrice et ledit procédé comprenant les étapes successives consistant à :

a. combiner l'émulsion pouvant être pompée avec l'extracteur dans des conditions de mélange ;

b. permettre la formation de produits d'encapsulation particulaires dans lesquels la phase dispersée est imbriquée dans une matrice solide du soluté formant une matrice ;

c. collecter les produits d'encapsulation et les séparer de l'extracteur.

Après d'importantes recherches, la demanderesse a découvert de façon surprenante et inattendue qu'il était possible d'atteindre les objectifs tels qu'énoncés précédemment en utilisant, dans une composition anhydre sous forme de poudre libre ou compacte, des particules comprenant un cœur contenant au moins un agent bénéfique et une enveloppe entourant le coeur; ladite enveloppe comprenant au moins un polysaccharide modifié hydrophobe et au moins un glucide hydrosoluble et/ou un polyol hydrosoluble; lesdites particules présentant simultanément une densité de poudre versée allant de 300,0g/l à 600,0g/l et une densité absolue supérieure à 1,0. Ces particules peuvent être en particulier obtenues par le procédé tel que décrit dans le brevet EP1917098B1 commenté précédemment.

Les particules conformes à la présente invention permettent d'encapsuler des ingrédients bénéfiques, en particulier fragiles, de façon complète (encapsulation totale) sans dégradation dans des capsules qui seraient suffisamment résistantes et étanches pour pouvoir être conservées sans altération à l'abri de l'humidité et pouvant être aisément formulées et rester stables dans des compositions anhydres sous forme de poudre. Ces mêmes particules dans ce type de composition présentent de préférence une morphologie sphérique et une densité de poudre versée très faible pour conserver la texture légère et douce ; elles ont également la capacité de s'ouvrir en présence d'eau pour pouvoir libérer leur agent bénéfique de façon quasi immédiate, progressive et répétable sur la peau, le cheveu et les phanères au contact de l'eau

Cette découverte est à la base de la présente invention.

La présente invention concerne une composition anhydre sous forme de poudre libre ou compacte comprenant :

1) au moins des particules comprenant un cœur contenant au moins un agent bénéfique et une enveloppe entourant le coeur; ladite enveloppe étant constitué d'au moins un polysaccharide modifié hydrophobe choisi parmi les $C_5$-$C_{20}$-alcényl succinates d'amidon et d'au moins un glucide hydrosoluble choisi parmi les ,maltodextrines ayant un dextrose équivalent allant de 4 à 20; et lesdites particules présentant simultanément une densité de poudre versée allant de 300,0g/l à 600,0g/l et une densité absolue supérieure à 1.0; les dites particules étant sphériques et ayant un diamètre moyen en nombre allant de 1 à 30$\mu$m, et un diamètre moyen en volume allant de 5 à 150$\mu$m; et

2) éventuellement au moins une charge autre que lesdites particules.

De préférence, la composition comprend un milieu physiologiquement acceptable.

Selon une forme particulière de l'invention, les compositions sont cosmétiques ou dermatologiques.

Selon une forme particulière de l'invention, les compositions selon l'invention peuvent être utilisées dans d'autres applications industrielles et notamment être des produits de consommation choisis parmi des produits à usage vétérinaire notamment des produits d'hygiène et/ou le soin comme les litières pour animaux; des produits d'entretien ménager comme les produits pour le soin et/ou le nettoyage du linge (lessives, adoucissants), les produits de vaisselle, les produits de nettoyage et/ou d'entretien des appareils électroménagers, les produits de nettoyage et/ou d'entretien des sols, carrelages, bois etc; des produits sanitaires comme des désodorisants, des produits détartrants, des produits déboucheurs de canalisations; des produits issus de l'industrie agro-alimentaire; des produits issus de l'agriculture; des produits phyto-sanitaires.

L'invention concerne également un procédé cosmétique de soin et/ou d'hygiène et/ou de de conditionnement et/ou de parfumage et/ou de maquillage d'une matière kératinique consistant à appliquer sur ladite matière kératinique une composition telle que définie précédemment.

L'invention concerne encore un procédé de traitement cosmétique des odeurs corporelles et éventuellement de la transpiration humaine consistant à appliquer sur une matière kératinique une composition telle que définie précédemment comprenant au moins un actif déodorant et/ou un actif anti-transpirant sous forme libre et/ou sous forme encapsulée.

L'invention concerne aussi un produit de consommation, caractérisé par le fait qu'il est constitué par une composition telle que définie précédemment.

**Définitions**

Par «anhydre», on entend au sens de la présente invention, une phase liquide présentant une teneur en eau inférieure à 5% en poids, de préférence inférieure à 2% en poids et de manière encore plus préférée inférieure à 1% en poids par rapport au poids de ladite composition voire encore moins de 0,5% et notamment exempte d'eau, l'eau n'étant pas ajoutée lors de la préparation de la composition mais correspondant à l'eau résiduelle apportée par les ingrédients mélangés

Au sens de la présente invention, on entend désigner :

- par «poudre compacte», une masse de produit dont la cohésion est assurée au moins en partie grâce à un compactage pendant la fabrication. En particulier, en réalisant une mesure par un texturomètre TA.XT.plus Texture Analyser vendu par la société Stable Micro Systems, la poudre compacte selon l'invention peut avantageusement présenter une résistance à la pression comprise entre 0.1 et 1kg, notamment entre 0.2 et 0.8kg, ramenée à la surface du mobile utilisé (en l'occurrence 7.07mm$^2$). La mesure de cette résistance est réalisée en faisant déplacer un mobile cylindrique à bout plat SMS P/3 au contact de la poudre, sur une distance de 2mm et à une vitesse de 0.5mm/seconde ; plus généralement cette poudre peut être obtenue par compactage, ou de préférence par pressage, Par «poudre compacte» il convient plus précisément d'entendre que ces poudres présentent une dureté shore A mesurée au moyen d'un duromètre ZWICK, qui varie, selon l'intensité des teintes considérées, de 12 à 30° Shore A.
- par «poudre libre», une masse de produit qui est apte à s'effondrer sous son propre poids; une telle masse étant formée de particules majoritairement isolées et mobiles les unes par rapport aux autres.

Au sens de la présente invention, on entend désigner par «milieu physiologiquement acceptable», un milieu convenant à l'administration d'une composition par voie topique. Un milieu physiologiquement acceptable est sans odeur désagréable et/ou aspect désagréable, et qui est parfaitement compatible avec la voie d'administration topique.

Par "matière kératinique", on entend la peau, le cuir chevelu, les lèvres, et/ou les phanères tels que les ongles et les fibres kératiniques, telles que par exemple, les poils, les cils, les sourcils et les cheveux.

Par «composition cosmétique», on entend au sens de l'invention toute composition appliquée sur une matière kératinique pour produire un effet non-thérapeutique d'hygiène, de soin, de conditionnement ou de maquillage contribuant à l'amélioration du bien-être et/ou à l'embellissement et/ou la modification de l'aspect de la matière kératinique sur laquelle on applique ladite composition.

Par «composition dermatologique», on entend au sens de l'invention toute composition appliquée sur une matière kératinique pour prévenir et/ou traiter un désordre ou un dysfonctionnement de ladite matière kératinique.

Par «traitement cosmétique», on entend au sens de l'invention tout effet non-thérapeutique de parfumage, d'hygiène, de soin, de conditionnement ou de maquillage contribuant à l'amélioration du bien-être et/ou à l'embellissement et/ou la modification de l'aspect ou de l'odeur de la matière kératinique sur laquelle on applique ladite composition.

Par «produit de consommation», on entend tout produit fabriqué destiné à être utilisé ou consommé dans la forme où il est commercialisé et qui n'est pas destiné à une fabrication ou modification ultérieure. Sans que les exemples soient limitatifs, les produits de consommation selon l'invention peuvent être des produits cosmétiques pour le soin et/ou l'hygiène et/ou le maquillage. de la peau, des lèvres, des ongles, des cils, des sourcils, des cheveux ou du cuir chevelu; des produits dermatologiques; des produits pour le soin des bébés comme les talcs; des produits pharmaceutiques; des produits à usage vétérinaire notamment des produits d'hygiène et/ou le soin comme les litières pour animaux; des produits d'entretien ménager comme les produits pour le soin et/ou le nettoyage du linge (lessives, adoucissants), les produits de vaisselle notamment pour les lave-vaisselles; les produits de nettoyage et/ou d'entretien des appareils électroménagers; les produits de nettoyage et/ou d'entretien des sols, carrelages, bois etc; des produits sanitaires comme des désodorisants, des produits détartrants, des produits déboucheurs de canalisations ; des produits issus de l'industrie agro-alimentaire; des produits issus de l'agriculture; des produits phytosanitaires.

Par «agent bénéfique», on entend au sens de l'invention tout composé présent dans un produit de consommation produisant un effet bénéfique perçu par le consommateur lors de son utilisation et/ou obtenu sur le produit de consommation lui-même, ledit effet bénéfique pouvant être une amélioration sensorielle ou une modification notamment visuelle et/ou olfactive et/ou tactile, une amélioration du confort et /ou de la facilité d'application, un effet esthétique, un effet hygiénique, une sensation de propreté, un effet curatif et/ou prophylactique.

Par «particules comprenant un cœur contenant au moins un agent bénéfique», on entend une particule comprenant au moins un agent bénéfique immobilisé, capturé et/ou encapsulé dans la matrice d'un système d'encapsulation ou de piégeage; ledit agent bénéfique étant libéré vers l'extérieur au fur et à mesure de la détérioration du système d'encapsulation ou de piégeage lorsque sa dégradation se produit au contact d'un milieu avec lequel il va réagir ou sous l'effet d'un stimulus tel qu'un apport d'eau.

**Densité de poudre versée (ou densité apparente non tassée)**

La détermination est effectuée à température ambiante (20-25°C) et dans les conditions normales atmosphériques (1 atmosphère) à l'aide d'une éprouvette de 100ml. L'éprouvette est pesée vide puis remplie d'un volume de 100ml de poudre versée, sans tassement. La différence de masse entre éprouvette vide et remplie de 100ml de poudre donne la densité de poudre versée.

**Densité absolue**

Principe de la mesure

La mesure consiste à déterminer le poids d'un échantillon du solide en poudre par simple pesée puis à mesurer le volume occupé par les particules de poudre en mesurant le volume de liquide déplacé par l'échantillon de poudre par immersion dans ce liquide. Le liquide choisi doit être peu volatil et ne doit pas être un solvant de la poudre. On choisit généralement le cyclohexane. Les mesures sont réalisées au moins deux fois.
Matériels : Une fiole jaugée de 10 ou 25ml et une balance de précision.

- $m_1$ est le poids de la fiole vide

- $m_2$ est le poids de la fiole remplie d'eau jusqu'au trait de jauge.

- $m_3$ est le poids de la fiole remplie de cyclohexane jusqu'au trait de jauge.

- $m_4$ est le poids de la fiole remplie environ au tiers de sa capacité par la poudre à analyser.

On remplit la fiole environ au tiers de sa capacité avec la poudre à analyser.

Méthode

On complète la fiole, un peu en-dessous du trait de jauge avec du cyclohexane. Afin d'éliminer complètement l'air emprisonné dans la poudre on opère comme suit :

    1) on traite la fiole dans un bac à ultra-sons pendant 5 minutes
    2) on ajuste le niveau du cyclohexane jusqu'au trait de jauge
    3) on traite la fiole dans un bac à ultra-sons pendant 2 minutes
    4) les étapes 2 et 3 sont répétées si nécessaire jusqu'à ce que le niveau du cyclohexane n'évolue plus.

$m_5$ est le poids de la fiole ainsi remplie.
Le poids de poudre analysée est égal à $m_4$-$m_1$ (pour une bonne précision ce poids doit être supérieur à 2g). La densité de l'air étant très faible par rapport à celle du solide on admet que $m_4$ - $m_1$ est égal au poids du solide constitutif de la poudre.
Le poids de cyclohexane correspondant au volume occupé par le solide (Vs) est égal à :

$$m_6 = (m_3 - m_1) - (m_5 - m_4) = \rho_{cyclo}.Vs$$

où $\rho_{cyclo}$ est la densité du cyclohexane à la température du laboratoire.
La densité absolue du solide constitutif de la poudre est égale à

$$\rho_{cyclos} = (m_4 - m_1)/Vs = \rho_{cyclo}(m_4 - m_1) / m_6.$$

Si la densité du cyclohexane à la température du laboratoire n'est pas connue, on la détermine comme suit par rapport à celle de l'eau :
Soit Vf le volume jaugé de la fiole et $\rho_{eau}$ la densité de l'eau à la température du laboratoire on a :

$$\rho_{cyclo} = (m_3 - m_1) / Vf \text{ et } \rho_{eau} = (m_2 - m_1) / Vf$$

soit

$$\rho_{cyclo} = \rho_{eau} (m_2 - m_1) / (m_3 - m_1)$$

La densité absolue du solide constitutif de la poudre est égale à :

$$\rho_s = [\rho_{eau} (m_4 - m_1) (m_2 - m_1)] / [m_6 (m_3 - m_1)].$$

## PARTICULES D'ENCAPSULATION

Les particules conformes à l'invention comprennent un cœur contenant au moins un agent bénéfique et une enveloppe entourant le cœur; ladite enveloppe étant constituée d'au moins un polysaccharide modifié hydrophobe choisi parmi les $C_5$-$C_{20}$-alcényl-succinates d'amidon et d'au moins un glucide hydrosoluble choisi parmi les maltodextrines ayant un dextrose équivalent allant de 4 à 20; lesdites particules présentant simultanément une densité de poudre versée allant de 300,0g/l à 600,0g/l et une densité absolue supérieure à 1,0.

Les particules conformes à la présente invention sont sphériques.

Par «sphériques», on entend que la particule présente un indice de sphéricité, c'est-à-dire le rapport entre son plus grand diamètre et son plus petit diamètre, est inférieur à 1,2. Dans ce cas, de telles particules sont généralement appelées «capsules».

Par « taille moyenne » des particules on entend les paramètres D[4,3] et D[2,3] mesurés en voie sèche par diffraction laser à l'aide d'un granulomètre Microtrac S3500, les résultats étant exprimés sous la forme de distributions granulométriques en volume et en nombre donnant accès au diamètre moyen.

Les particules sphériques conformes à la présente invention présentent, ainsi un diamètre moyen en nombre allant de 1 à 30$\mu$m, plus préférentiellement allant de 2 à 15$\mu$m et encore mieux de 5 à 10$\mu$m et un diamètre moyen en volume allant de 5 à 150$\mu$m, de préférence allant de 10 à 100$\mu$m et encore mieux de 20 à 80$\mu$m.

Les particules selon l'invention contenant l'agent bénéfique représentent, de préférence, de 0,1 à 60% en poids, de préférence 0,3 à 40% en poids et encore mieux de 0,5 à 20% en poids du poids total de la composition.

## POLYSACCHARIDE MODIFIE HYDROPHOBE

On entend par «polysaccharide modifié hydrophobe» tout polysaccharide modifié par voie chimique ou enzymatique et comportant au moins un groupe fonctionnel hydrophobe.

Les polysaccharides sont des macromolécules glucidiques formées par l'enchaînement d'un grand nombre de sucres élémentaires (oses) hydrophiles liés entre eux par des liaisons O-osidiques.

Les groupes fonctionnels hydrophobes sont des groupes hydrocarbonés (constitués essentiellement d'atomes de carbone et d'hydrogène) comprenant au moins 4 atomes de carbone, de préférence au moins 6 et encore mieux au moins 8 atomes de carbone tels que des groupes alkyles, alcényles, aryles (ie: phényle) ou aralkyles (ie: benzyle). Le nombre maximum d'atomes de carbone du groupe hydrocarboné est, de préférence, 24, plus préférentiellement 20, et encore plus préférentiellement 18. Les groupes hydrocarbonés hydrophobes peuvent être non substitués, par exemple constitués d'une simple longue chaine alkyle ou bien substitués par des groupes non réactifs comme des groupes aromatiques tels que des groupes aryles (ie: phényle) ou aralkyles (ie: benzyle) ou encore des groupes polaires tels que par exemple des carboxyles ou des hydroxyles.

Pour greffer le ou les groupe(s) fonctionnel(s) hydrophobe(s) sur les polysaccharides, on utilise généralement des dérivés halogénés, des époxydes, des isocyanates, des acides carboxyliques ou leurs dérivés (esters, halogénures d'acides, anhydrides)

Parmi les polysaccharides modifiés hydrophobes il a les polysaccharides neutres modifiés hydrophobes tels que:

-   les celluloses et leurs dérivés en particulier méthyl-, hydroxyéthyl-, éthylhydroxyéthyl-, hydroxypropyl-, hydroxypropylméthyl-, carboxyméthyl-cellulose modifiés hydrophobes. Les groupements hydrophobes préférés sont choisis parmi les radicaux alkyles en $C_8$-$C_{18}$ et plus particulièrement les radicaux alkyles en $C_{12}$-$C_{18}$. En particulier, les polysaccharides neutres modifiés hydrophobes désignent l'éthylhydroxyéthylcellulose ou l'hydroxyéthylcellulose modifiés hydrophobes et notamment ceux commercialisés par Ashland sous la dénomination commerciale Natrosol Plus;

- les amidons et leurs dérivés (en particulier: hydroxyéthyl-, hydroxypropyl-, carboxyméthyl-amidon) modifiés hydrophobes ainsi que les amidons dégradés et/ou estérifiés modifiés hydrophobes,
- les dextranes modifiés hydrophobes tels que notamment les phénoxy-dextranes obtenus par réaction entre le 1,2-époxy-3-phénoxypropane et un dextrane; les $C_6$-$C_{12}$ alkyl-dextranes obtenus par réaction entre les 1,2-époxy-$C_6$-$C_{12}$ alcanes tels que le 1,2-époxyoctane ou le 1,2-époxydodecane et un dextrane;
- les guars et leurs dérivés hydroxyéthyl-, carboxyméthyl- et hydroxypropyl- guars modifiés hydrophobe ;
- les pullulanes modifiés hydrophobes tels que les cholestérylpullulanes;
- les inulines modifiées hydrophobes via des fonctions les éthers, esters et carbamates d'alkyle, en particulier les carbamates à chaînes alkyles en $C_4$-$C_{18}$ et plus particulièrement ceux commercialisés sous l'appellation Inutec® SP1.

Le polysaccharide modifié hydrophobe représente de préférence de 20 à 90% en poids, notamment 30 à 80% en poids, mieux 40 à 70% en poids, et encore mieux 40 à 60% en poids par rapport au poids total de l'enveloppe de la particule.

Selon l'invention, on choisira parmi les polysaccharides modifiés hydrophobes les amidons modifiés hydrophobes.

Les molécules d'amidons peuvent avoir comme origine botanique les céréales ou encore les tubercules. Ainsi, les amidons sont par exemple choisis parmi les amidons de maïs, de riz, de manioc, de tapioca, d'orge, de pomme de terre, de blé, de sorgho, de pois.

On entend par «amidon modifié hydrophobe» tout amidon modifié par réaction chimique ou enzymatique et comprenant au moins un groupe fonctionnel hydrophobe.

Les amidons modifiés hydrophobes conformes à l'invention sont choisis parmi les $C_5$-$C_{20}$ alcényl succinates d'amidon, plus particulièrement les $C_5$-$C_{20}$-alcényl succinates et encore mieux l'octényle succinate d'amidon sodique (E1450- CAS 66829-29-6 / 52906-93-1/70714-61-3), en particulier celui commercialisé par National Starch sous la dénomination CAPSUL®.

On peut également citer les références commerciales CAPSUL TA®, N-LOK®, N- LOK 1930®, HI-CAP 100®, PURITY GUM 1773®, PURITY GUM 2000 ® de National Starch, CLEARGUM CO® de la société Roquette et EMCAP®, EMTEX®, DELITEX de la société Cargill.

## GLUCIDE OU POLYOL HYDROSOLUBLE

Par «glucide hydrosoluble » ou «polyol hydrosoluble», on entend un glucide ou un polyol qui, introduit dans l'eau sans modification de pH à 25°C, à une concentration massique égale à 3%, permet l'obtention d'une solution macroscopiquement homogène et transparente, c'est à dire ayant une valeur de transmittance minimum de la lumière, à une longueur d'onde égale à 500nm, à travers un échantillon de 1cm d'épaisseur, d'au moins 80%, de préférence d'au moins 90%.

Par «glucides» (encore appelés hydrates de carbone ou carbohydrates ou saccharides) on entend l'ensemble des sucres simples ou oses et leurs combinaisons ou osides.

Les glucides comprennent habituellement:

(1) les monosaccharides ou oses qui sont de deux types: les aldoses comprenant une fonction aldéhyde sur le premier carbone et les cétoses comprenant une fonction cétone sur le deuxième carbone. On les distingue aussi suivant le nombre d'atomes de carbone qu'ils possèdent.

(2) les oligosaccharides (ou oligosides) qui sont des oligomères d'oses ayant un enchaînement de 2 à 10 unités monosaccharides unies par des liaisons glycosidiques.

(3) Les polyholosides (ou polysaccharides ou polyosides) qui sont des polymères d'oses ayant un enchaînement de monosaccharides supérieur à 10 unités

## GLUCIDES HYDROSOLUBLES

### (1) Les oses ou monosaccharides

Parmi les oses ou monosaccharides utilisables, on peut citer, seuls ou en mélanges

- les tétroses possédant 4 carbones: érythrose, thréose, érythrulose;
- les pentoses possédant 5 carbones: ribose, arabinose, xylose, désoxyribose;
- les hexoses possédant 6 carbones: glucose, mannose, fucose, gulose, idose, galactose, talose, fuculose, fructose, sorbose, le rhamnose;

- les heptoses possédant 7 carbones: sédoheptulose sous leur forme D et/ou L.

Parmi les monosaccharides, on peut citer l'arabinose, le xylose, le fructose, le glucose, le mannose, le rhamnose, le thréose.

(2) Les oligosaccharides

Parmi les oligosaccharides utilisables selon l'invention, on peut citer

(i) Les disaccharides ou diholosides ou diosides composés de deux molécules d'ose.
Parmi les disaccharides on peut citer: le cellobiose, l'isomaltose, l'isomaltulose, le lactose, le lactulose, le maltose, le saccharose, le tréhalose ou le mélibiose

(ii) Les triholosides composés de trois molécules d'oses tels que par exemple: le raffinose ou le maltotriose.

(iii) les dextrines qui sont des mélanges d'oligosides de glucose linéaires dont les unités de glucose sont liées par des liaisons osidiques du type $\alpha$-(1,4) ou $\alpha$-(1,6).

(iv) les sirops de glucose obtenus par hydrolyse acide ou enzymatique de l'amidon dont le D.E. est compris entre 20 et 100. D.E. ou «dextrose équivalent», est l'indicateur du degré d'hydrolyse de l'amidon. Plus le D.E. est élevé, plus l'hydrolyse est poussée, et donc plus la proportion en sucres simples (à chaîne courte) est élevée.

(v) les sirops de glucose-fructose notamment à haute teneur en fructose (HFCS : high-fructose corn syrup) qui désignent une série de sirops de maïs qui ont été soumis à des processus enzymatiques en vue d'augmenter leur teneur en fructose avant d'être mélangés à du sirop de glucose pour obtenir leur composition finale.

Parmi les sirops de glucose-fructose également dénommés sirops d'isoglucose on peut citer :

- le HFCS 90, qui contient 90% de fructose et 10% de sirop de glucose.
- le HFCS 55, qui contient 55% de fructose et 45% de sirop de glucose.
- le HFCS 42, qui contient 42% de fructose et 58% de sirop de glucose.

Parmi les oligosaccharides, on utilisera plus préférentiellement le cellobiose, le maltose, l'isomaltose, le raffinose, les sirops de glucose, plus particulièrement les sirops de glucose.
On peut citer également un sirop de glucose de D.E. compris allant de 21 à 60 et encore plus préférentiellement un sirop de glucose de D.E. compris de 21 à 38 tels que par exemple les sirops de glucose déshydratés commercialisés par Téreos sous les dénominations G210, G290 et G380

(3) les polysaccharides ou polyholosides

On peut citer par exemple

- les dextranes qui sont composés d'unités D-glucose reliées par un liaison osidique $\alpha(1{\rightarrow}6)$ et possèdent des ramifications constituées de liaisons alpha-1,2 ou 1,3 ou 1,4. Ils sont préparés par fermentation du sucre de betterave contenant uniquement des groupements hydroxyles. Il est possible d'obtenir à partir du dextrane natif par hydrolyse et purification, des fractions de dextrane de poids moléculaires différents. Le dextrane peut en particulier se présenter sous la forme de sulfate de dextrane.

- les pullulanes qui sont constitués d'unités maltotriose, connues sous le nom d'$\alpha$-(1,4)-$\alpha$(1,6)-glucane. Trois unités de glucose dans le maltotriose sont connectées par une liaison glycosidiques en $\alpha$-(1,4), tandis que les unités maltotriose consécutives sont connectées l'une à l'autre par une liaison glycosidique en $\alpha$-(1,6). Il est produit à partir de l'amidon par le champignon Aureobasidium pullulans. Le pullulane est par exemple produit sous la référence PULLULAN PF 20® par le groupe Hayashibara au Japon.

- les maltodextrines qui sont le résultat de l'hydrolyse d'un amidon (ie : blé, maïs) ou d'une fécule (ie : pomme de terre). Elles sont constituées de différents sucres (ie : glucose, maltose, maltotriose, oligosides et polyosides) directement issus de cette réaction, dans des proportions qui dépendent du degré de l'hydrolyse.

Ce degré est mesuré par « dextrose équivalent », ou D.E., le dextrose ou D-glucose étant le résultat d'une hydrolyse totale de l'amidon. Plus le D.E. est élevé, plus l'hydrolyse est poussée, et donc plus la proportion en sucres simples (à chaîne courte) composant la maltodextrine est élevée.

Les maltodextrines utilisées conformément à l'invention sont de D.E. allant de 4 à 20 et encore mieux les maltodextrines de D.E. allant de 12 à 20.

On utilisera de préférence les maltodextrines de pomme de terre ou de maïs telles que celles vendues sous les dénominations commerciales MD 20P® de AVEBE, MALDEX 120®, MALDEX 170®, MALDEX 190® de Tereos.

**POLYOLS**

Les polyols, sont des composés carbonés, notamment hydrocarbonés, linéaires, ramifiés et/ou cycliques, non glycosidiques , saturés ou insaturés, comprenant 4 à 18 atomes de carbone, notamment 4 à 16, voire 4 à 12 atomes de carbone, et 3 à 9 groupes hydroxy (OH), et pouvant comprendre en outre un ou plusieurs atomes d'oxygène intercalés dans la chaîne (fonction éther).

Les polyols sont des composés hydrocarbonés saturés, linéaires ou ramifiés, comprenant 4 à 18 atomes de carbone, notamment 4 à 16, voire 4 à 12 atomes de carbone, et 3 à 9 groupes hydroxy (OH).

Ils peuvent être choisis, seuls ou en mélanges, parmi:

- les triols, tels que le triméthyloléthane ou le triméthylolpropane
- les tétraols, tels que le pentaérythritol (tétraméthylolméthane), l'érythritol, le diglycérol ou le ditriméthylolpropane;
- les pentols tels que l'arabitol ;
- les hexols tels que le dulcitol, le sorbitol, le mannitol, le dipentaérythritol ou le triglycérol ;
- les heptols tels que le volémitol ;
- les octaols ;
- les nonanols tels que l'isomalt, le maltitol, l'isomaltitol ou le lactilol.

Parmi les glucides hydrosolubles on cite les dextranes, les pullulanes, les sirops de glucose et les maltodextrines et encore mieux les sirops de glucose de D.E. allant de 21 à 38 et/ou les maltodextrines de D.E. allant de 4 à 20 et encore mieux les maltodextrines de D.E. allant de 12 à 20.

On cite les sirops de glucose tels que ceux vendus par Tereos sous les dénominations G210, G290 et G380 et les maltodextrines de pomme de terre ou de maïs telles que celles vendues sous les dénominations commerciales MD 20P® de AVEBE. MALDEX 120®, MALDEX 170®, MALDEX 190® de Tereos.

Les glucides hydrosoluble(s) conformes à l'invention représentent de 10 à 80% en poids, de préférence de 15 à 70% en poids, plus préférentiellement de 20 à 65% en poids, et encore mieux de 40 à 60% en poids par rapport au poids total de l'enveloppe de la particule.

Selon une forme particulièrement préférée de l'invention, l'enveloppe des particules selon l'invention est constituée

- d'au moins un $C_5$-$C_{20}$-alcényl succinate d'amidon et
- d'au moins une maltodextrine de D.E. allant de 4 à 20 et de préférence allant de 12 à 20 et/ou un sirop de glucose de D.E. allant de 21 à 60, préférentiellement de 21 à 38.

Selon une première variante, l'enveloppe des particules selon l'invention est constituée d'au moins un $C_5$-$C_{20}$-alcényl succinate d'amidon et d'au moins une maltodextrine de D.E. allant de 4 à 20 et de préférence allant de 12 à 20.

Selon une deuxième variante l'enveloppe des particules selon l'invention est constituée d'au moins un $C_5$-$C_{20}$-alcényl succinate d'amidon et d'au moins un sirop de glucose de D.E. allant de 21 à 60, préférentiellement de 21 à 38.

Selon une forme particulièrement préférée de l'invention, l'enveloppe des particules d'encapsulation est constituée

a) d'au moins un $C_5$-$C_{20}$-alcényl succinate d'amidon dans une quantité allant de 20 à 90% en poids, notamment 30 à 80% en poids, de préférence 40 à 70% et encore mieux 40 à 60% en poids, par rapport au poids total de l'enveloppe de la particule.

b) et une maltodextrine de D.E. allant de 4 à 20 dans une quantité allant de 10 à 80% en poids, de préférence de 15 à 70% en poids, plus préférentiellement de 20 à 65% en poids, et encore mieux de 40 à 60% en poids par rapport au poids total de l'enveloppe de la particule.

Selon une forme particulièrement préférée de l'invention, l'enveloppe des particules d'encapsulation est constituée

a) d'au moins un $C_5$-$C_{20}$-alcényl succinate d'amidon dans une quantité allant de 20 à 90% en poids, notamment 30 à 80% en poids, de préférence 40 à 70% et encore mieux 40 à 60% en poids, par rapport au poids total de l'enveloppe de la particule.

b) et d'au moins une maltodextrine de D.E. allant de 4 à 20 dans une quantité allant de 10 à 80% en poids, de préférence de 15 à 70% en poids, plus préférentiellement de 20 à 65% en poids, et encore mieux de 40 à 60% en poids par rapport au poids total de l'enveloppe de la particule.

**Procédé de préparation des particules à libération d'agent bénéfique**

Les particules selon l'invention peuvent notamment être préparées selon le procédé décrit dans le brevet EP1917098B1 de FeyeCon.

Selon une forme particulière de l'invention, les particules sont obtenues selon un procédé comprenant au moins les étapes suivantes :

- on prépare une solution aqueuse constituée du mélange du glucide hydrosoluble et/ou du polyol hydrosoluble et du polysaccharide modifié hydrophobe puis on ajoute l'agent bénéfique et on agite de façon à former une émulsion ; et
- on homogénéise ladite émulsion ainsi formée sous haute pression à une pression allant de 10 à 200 bars et plus préférentiellement de 20 à 200 bars; et
- on pulvérise, de préférence en continu, ladite émulsion dans une chambre de séchage; et
- l'eau est extraite pendant une durée, de préférence, ne dépassant pas 3 heures, et plus préférentiellement ne dépassant pas 30 minutes, avec un fluide sous pression tel que le dioxyde de carbone, de préférence à l'état supercritique, de préférence à une pression d'au moins 0,3XPc et à une température d'au moins Tc-60°C avec Pc correspondant à la pression critique du gaz et Tc la température critique du gaz, de façon à obtenir des particules, de préférence sphériques, de taille moyenne, de préférence allant de 1 à 150$\mu$m, plus préférentiellement allant de 2 à 100$\mu$m et encore mieux de 5 à 80$\mu$m.

**CHARGES**

Par «charge», il faut comprendre toute particule minérale ou organique non colorante, sous forme libre (non encapsulée), autre que les particules selon l'invention telles que définies précédemment. Lesdites charges sont de préférence solides à température allant de 20 à 35°C et de toute forme.

Les charges utilisées dans les compositions selon la présente invention peuvent être de formes lamellaires (ou plaquettaires), sphériques (ou globulaires), de fibres ou de toute autre forme intermédiaire entre ces formes définies. De préférence, la composition selon la présente invention comporte majoritairement, voire exclusivement, des charges lamellaires.

La ou les charges peuvent être présentes à une teneur allant de 20 à 99,9 % en poids, de préférence de 40 à 99,8% en poids, plus préférentiellement encore de 60 à 99% en poids, par rapport au poids total de la composition.

La (ou les) charge(s) avantageusement utilisée(s) dans une composition selon l'invention peuvent être de formes lamellaires (ou plaquettaires), sphériques (ou globulaires), de fibres ou de toute autre forme intermédiaire entre ces formes définies.

De préférence, la composition selon la présente invention comporte majoritairement, voire exclusivement, des charges lamellaires.

Ces charges selon l'invention peuvent être ou non enrobées superficiellement, et, en particulier, elles peuvent être traitées en surface par des silicones, des acides aminés, des dérivés fluorés ou toute autre substance favorisant la dispersion et la compatibilité de la charge dans la composition.

De préférence, une composition selon l'invention comprend au moins une charge additionnelle minérale. De préférence, cette (ou ces) charge(s) minérale(s) est (ou sont) choisie(s) parmi le talc, le mica, la silice, les silicate de magnésium et d'aluminium, le siloxysilicate de triméthyle, le kaolin, la bentone, le carbonate de calcium et l'hydrogéno-carbonate de magnésium, l'hydroxyapatite, le nitrure de bore, les microsphères de silice creuses (Silice Beads de Maprecos), les microcapsules de verre ou de céramique, les charges à base de silice comme l'Aerosil 200, l'Aerosil 300 ; la Sunsphere H-33, la Sunsphere H-51 commercialisés par Asahi Glass ; le Chemicelen commercialisé par Asahi Chemical ; les composites de silice et de dioxyde de titane, comme la série TSG commercialisée par Nippon Sheet Glass, la fluorphlogopite, et leurs mélanges.

De préférence, une composition selon l'invention comprend au moins une charge additionnelle organique. De préférence, cette (ou ces) charge(s) organique(s) minérale est (ou sont) choisie(s) parmi les poudres de polyamide (Nylon® Orgasol de chez Atochem), de poly-b-alanine et polyéthylène, les poudres de polytétrafluoroéthylène (Téflon®), la lauroyl-lysine, l'amidon, les poudres d'amidon estérifié par l'anhydride octénylsuccinique ou l'anhydride dodécyl succinique , commercialisées sous la dénomination DRY-FLO® par la société National Starch, les poudres de polymères de tétrafluoroéthylène, les microsphères creuses de polymères, par exemple comprenant un (alkyl)acrylate, telles l'Expancel® (Nobel Industrie), les savons métalliques dérivés d'acides organiques carboxyliques ayant de 8 à 22 atomes de carbone, de préférence, de 12 à 18 atomes de carbone, par exemple, le stéarate de zinc, de magnésium ou de lithium, le laurate de zinc, le myristate de magnésium, le Polypore® L 200 (Chemdal Corporation), les microbilles de résine de silicone (Tospearl® de Toshiba, par exemple), les poudres de polyuréthane, en particulier, les poudres de polyuréthane réticulé comprenant un copolymère, ledit copolymère comprenant du triméthylol hexyllactone, comme le polymère d'hexaméthylène di-isocyanate/triméthylol hexyllactone, commercialisé sous la dénomination de PLASTIC POWDER D-400® ou PLASTIC POWDER D-800® par la société TOSHIKI, les microcires de Carnauba, telles que celle commercialisée sous la dénomination de MicroCare 350® par la société MICRO POWDERS, les microcires de cire synthétique, telles que celle commercialisée sous la dénomination de MicroEase 114S® par la société MICRO POWDERS, les microcires constituées d'un mélange de cire de Carnauba et de cire de polyéthylène, telles que celles commercialisées sous les dénominations de Micro Care 300® et 310® par la société MICRO POWDERS, les microcires constituées d'un mélange de cire de Carnauba et de cire synthétique, telles que celle commercialisée sous la dénomination Micro Care 325® par la société MICRO POWDERS, les microcires de polyéthylène, telles que celles commercialisées sous les dénominations de Micropoly 200®, 220®, 220L® et 250S® par la société MICRO POWDERS ; les fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser. les fibres ont une longueur allant de $1\mu m$ à 10mm, de préférence de 0,1mm à 5mm et mieux de 0,3mm à 3mm. Leur section peut être comprise dans un cercle de diamètre allant de 2nm à $500\mu m$, de préférence allant de 100nm à $100\mu m$ et mieux de $1\mu m$ à $50\mu m$. A titre de fibres utilisables dans les compositions selon l'invention, on peut citer les fibres non rigides telles que les fibres de polyamide (Nylon®) ou les fibres rigides telles que les fibres de polyimide-amide comme celles vendues sous les dénominations KERMEL®, KERMEL TECH® par la société RHODIA ou de poly-(p-phénylène-téréphtalamide) (ou d'aramide) notamment vendues sous la dénomination Kevlar ® par la société DUPONT DE NEMOURS, et leurs mélanges.

A titre représentatif de telles charges mises en œuvre de façon préférée dans la cadre de la présente invention, peuvent notamment être citées : le talc, le mica, l'amidon, la fluorphlogopite, les argiles tels que le silicate d'aluminium et de magnésium, ou encore les microsphères creuses de polymère.

Selon une forme particulière de l'invention, la phase pulvérulente comprend de préférence en outre des agents de coloration.

## Agents de coloration

Selon une forme particulière de l'invention, la composition comprend au moins un agent de coloration sous forme libre et/ou sous forme encapsulée. Elle comprend de préférence plus largement au moins un agent de coloration choisi parmi las nacres, les pigments, les particules réfléchissantes, et leur(s) mélange(s).

Ladite composition présente avantageusement une teneur en agents de coloration allant de 0% à 40% en poids, mieux de 1 à 30% en poids, par rapport au poids total de la composition.

### Pigments

Par «pigments», il faut comprendre des particules de toute forme, blanches ou colorées, minérales ou organiques, insolubles dans le milieu physiologique, destinées à colorer la composition.

Les pigments peuvent être blancs ou colorés, minéraux et/ou organiques.

On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Les pigments organiques peuvent être choisis parmi les matériaux ci-dessous et leurs mélanges:

le carmin de cochenille,
les pigments organiques de colorants azoïques, anthraquinoniques, indigoïdes, xanthéniques, pyréniques, quinoliniques, de triphénylméthane, de fluorane.

Parmi les pigments organiques, on peut notamment citer les pigments certifiés D&C connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

Les matériaux chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.

Une composition selon l'invention peut comprendre une teneur en pigments allant de 0% à 30% en poids, par rapport au poids total de la composition, de préférence allant de 2% à 15% en poids, et préférentiellement allant de 4% à 20% en poids par rapport au poids total de la composition.

Nacres

Par «nacres», il faut comprendre des particules colorées de toute forme, irisées ou non, notamment produites par certains mollusques dans leur coquille ou bien synthétisées et qui présentent un effet de couleur par interférence optique.

Comme exemples de nacres, on peut citer les pigments nacrés tels que le mica titane recouvert avec un oxyde de fer, le mica recouvert d'oxychlorure de bismuth, le mica titane recouvert avec de l'oxyde de chrome, les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré. A titre illustratif des nacres pouvant être introduites dans la composition, on peut citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne); les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne); les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna); les nacres de teinte brune notamment commercialisées par la société ENGELHARD sous la dénomination Nu-antique copper 340XB (Cloisonne) et Brown CL4509 (Chromalite); les nacres à reflet cuivre notamment commercialisées par la société ENGELHARD sous la dénomination Copper 340A (Timica); les nacres à reflet rouge notamment commercialisées par la société MERCK sous la dénomination Sienna fine (17386) (Colorona); les nacres à reflet jaune notamment commercialisées par la société ENGELHARD sous la dénomination Yellow (4502) (Chromalite); les nacres de teinte rouge à reflet or notamment commercialisées par la société ENGELHARD sous la dénomination Sunstone G012 (Gemtone); les nacres roses notamment commercialisées par la société ENGELHARD sous la dénomination Tan opale G005 (Gemtone); les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Toujours à titre d'exemple de nacres, on peut citer également les particules comportant un substrat de borosilicate enrobé d'oxyde de titane.

Des particules à substrat de verre revêtu d'oxyde de titane sont notamment vendues sous la dénomination METASHINE MC1080RY par la société TOYAL.

Enfin, comme exemples de nacres, on peut également citer les paillettes de polyéthylène téréphthalate, notamment celles commercialisées par la société Meadowbrook Inventions sous le nom Silver 1P 0.004X0.004 (paillettes argentées).

Les compositions selon l'invention peuvent comprendre une teneur en nacres allant de 0% à 30% en poids, par exemple de 0.01 à 5% en poids, par rapport au poids total de la composition.

Particules réfléchissantes

Par «particules réfléchissantes», on désigne des particules dont la taille, la structure, notamment l'épaisseur de la ou des couches qui la constituent et leurs natures physique et chimique, et l'état de surface, leur permettent de réfléchir la lumière incidente. Cette réflexion peut, le cas échéant, posséder une intensité suffisante pour créer à la surface de la composition ou du mélange, lorsque celui-ci est appliqué sur le support à maquiller, des points de surbrillance visibles à l'œil nu, c'est-à-dire des points plus lumineux qui contrastent avec leur environnement en semblant briller.

Les particules réfléchissantes peuvent être sélectionnées de manière à ne pas altérer significativement l'effet de coloration généré par les agents de coloration qui leur sont associés et plus particulièrement de manière à optimiser cet effet en terme de rendu de couleur. Elles peuvent plus particulièrement posséder une couleur ou un reflet jaune, rose, rouge, bronze, orangé, brun, or et/ou cuivré.

Ces particules peuvent présenter des formes variées, notamment être en forme de plaquettes ou globulaires, en particulier sphériques. Les particules réfléchissantes, quelle que soit leur forme, peuvent présenter une structure multicouche ou non et, dans le cas d'une structure multicouche, par exemple au moins une couche d'épaisseur uniforme, notamment d'un matériau réfléchissant.

Lorsque les particules réfléchissantes ne présentent pas de structure multicouche, elles peuvent être composées par exemple d'oxydes métalliques, notamment des oxydes de titane ou de fer obtenus par synthèse.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant notamment d'au moins un métal ou matériau métallique. Le substrat peut être monomatière, multimatériau, organique et/ou inorganique.

Plus particulièrement, il peut être choisi parmi les verres, les céramiques, le graphite, les oxydes métalliques, les alumines, les silices, les silicates, notamment les aluminosilicates et les borosilicates, le mica synthétique et leurs mélanges, cette liste n'étant pas limitative.

Le matériau réfléchissant peut comporter une couche de métal ou d'un matériau métallique. Des particules réfléchissantes sont décrites notamment dans les documents JP-A-09188830, JP-A-10158450, JP-A-10158541, JP-A-07258460 et JP-A-05017710.

Toujours à titre d'exemple de particules réfléchissantes comportant un substrat minéral enrobé d'une couche de métal, on peut citer également les particules comportant un substrat de borosilicate enrobé d'argent.

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

On peut également utiliser des particules comprenant un substrat métallique tel que l'argent, l'aluminium, le fer, le chrome, le nickel, le molybdène, l'or, le cuivre, le zinc, l'étain, le magnésium, l'acier, le bronze, le titane, ledit substrat étant enrobé d'au moins une couche d'au moins un oxyde métallique tels que l'oxyde de titane, l'oxyde d'aluminium, l'oxyde de fer, l'oxyde de cérium, l'oxyde de chrome, les oxydes de silicium et leurs mélanges. On peut citer à titre d'exemple les poudres d'aluminium, de bronze ou de cuivre enrobées de $SiO_2$ commercialisées sous la dénomination VISIONAIRE par la société ECKART.

Les compositions selon l'invention peuvent comprendre une teneur en particules réfléchissantes allant de 0% à 30% en poids, par exemple de 0.01 à 5% en poids, par rapport au poids total de la composition.

De préférence, les agents de coloration sont choisis parmi :

- les pigments organiques avantageusement choisies parmi les pigments certifiés D & C par la Food & Drug Administration tels que répertoriés à la section « Color Additives - Batch Certified by the U.S. Food and Drug Administrationage » du CTFA ; on peut citer notamment les Blue 1 et 4, Brown 1, Ext.Violet 2, Ext.Yellow 7, les Green 3, 5, 6, 8, les Orange 4, 5, 10, 11, les Red 4, 6, 7, 17, 21, 22, 27, 28, 30, 36 et 40, le Violet 2, les Yellow 5, 6, 7, 8, 10 et 11, et leurs mélanges,
- les pigments minéraux avantageusement choisies parmi les oxydes de fer, de titane, de zirconium, de cérium, de zinc, de fer ou de chrome, le bleu ferrique, le violet de manganèse, le bleu, le rose ou le violet d'outremer, l'hydrate de chrome, l'hydroxyde de chrome, l'oxychlorure de bismuth, et leurs mélanges.

Selon une forme particulière de l'invention, la phase pulvérulente comprend de préférence en outre une phase huileuse.

## PHASE HUILEUSE

La composition selon l'invention peut avantageusement comprendre au moins une phase grasse qui peut comprendre au moins un composé choisi parmi les huiles, les cires et/ou les solvants d'origine minérale, animale, végétale ou synthétique, carbonés, hydrocarbonés, fluorés et/ou siliconés, volatils ou non volatils, seuls ou en mélange dans la mesure où ils forment un mélange homogène et stable et sont compatibles avec l'utilisation envisagée.

Par 'volatil', on entend au sens de l'invention, tout composé susceptible de s'évaporer au contact des matières kératiniques en moins d'une heure, à température ambiante (25°C) et pression atmosphérique (1atm). Notamment, ce composé volatil a une pression de vapeur non nulle, à température ambiante et pression atmosphérique, notamment allant de 0,13Pa à 40 000Pa ($10^{-3}$ à 300 mm de Hg), en particulier allant de 1,3Pa à 13 000Pa (0,01 à 100 mm de Hg), et plus particulièrement allant de 1,3Pa à 1300Pa (0,01 à 10mm de Hg).

Par opposition, on entend par 'non volatil', un composé restant sur les matières kératiniques humaines à température ambiante et pression atmosphérique, au moins une heure et ayant notamment une pression de vapeur inférieure à $10^{-3}$mm de Hg (0,13Pa).

## AGENTS BENEFIQUES

La quantité d'agent bénéfique présent dans les particules conformes à l'invention varie de préférence de 10 à 80% en poids du poids de la particule, de préférence 15 à 60% en poids et encore mieux 20 à 40% en poids du poids total de la particule.

Le temps de libération de l'agent bénéfique variera évidemment selon la nature et l'intensité du stimulus.

La durée totale pour libérer l'agent bénéfique pourra être modulée et sera fortement dépendante de la composition, de la teneur en particules, de la nature notamment chimique de l'agent bénéfique et de sa concentration dans les particules (quantité encapsulée dans la particule) et de la nature et de l'intensité du stimulus auquel sera soumise la particule contenant l'agent bénéfique. La libération peut aussi bien être quasi instantanée ou durer plusieurs heures voire plusieurs jours.

Parmi les agents bénéfiques utilisables selon l'invention, on peut citer plus particulièrement

(i) les corps gras;
(ii) les substances parfumantes;
(iii) les principes actifs pharmaceutiques;
(iv) les actifs cosmétiques.

### a) Corps gras

Ils peuvent être choisis dans le groupe comprenant

(i) les huiles naturelles d'origine végétale, animale ou marine
(ii) les huiles minérales ,
(iii) les huiles hydrogénées,
(iv) les huiles de silicone,
(v) les terpènes,
(vi) le squalène,
(vii) les acides gras saturés ou insaturés
(viii), les esters d'acide gras,
(x) les cires,
(x) les alcools gras,
(xi) les beurres comme le beurre de karité ou le beurre de cacao,
(xii) et leurs mélanges

### b) Les substances parfumantes

Par «substance parfumante», on entend tout ingrédient susceptible de dégager une odeur agréable.

Les parfums sont des compositions contenant notamment des matières premières (dénommées généralement ingrédients de parfumerie) qui sont décrites dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III.

Il peut s'agir de produits de synthèse ou de produits naturels, comme des huiles essentielles, des absolus, des résinoïdes, des résines, des concrètes, et/ou des produits synthétiques (hydrocarbures terpéniques ou sesquiterpéniques, alcools, phénols, aldéhydes, cétones, éthers, acides, esters, nitriles, peroxydes, saturés ou insaturés, aliphatiques ou cycliques).

Selon la définition donnée dans la norme internationale ISO 9235 et adoptée par la Commission de la Pharmacopée Européenne, une huile essentielle est un produit odorant généralement de composition complexe, obtenu à partir d'une matière première végétale botaniquement définie, soit par entraînement à la vapeur d'eau, soit par distillation sèche, soit par un procédé mécanique approprié sans chauffage. L'huile essentielle est le plus souvent séparée de la phase aqueuse par un procédé physique n'entraînant pas de changement significatif de la composition.

Parmi les huiles essentielles utilisables selon l'invention, on peut citer celles obtenues à partir des plantes appartenant aux familles botaniques suivantes :

Abiétaceés ou Pinacées: conifères; Amaryllidacées; Anacardiacées; Anonacées: ylang; Apiacées (par exemple les ombellifères): aneth, angélique, coriandre, criste marine, carotte, persil; Aracées; Aristolochiacées; Astéracées: achilée, armoise, camomille, hélichryse; Bétulacées; Brassicacées; Burséracées: encen ; Caryophyllacées; Canellacées; Césalpiniacées: copaïfera (copahu); Chénopodacées; Cistacées: ciste; Cypéracées; Diptérocarpacées ; Ericacées: gaulthérie (wintergreen); Euphorbiacées ; Fabacées ; Geraniacées : géranium ; Guttifères ; Hamamélidacées ; Hernandiacées; Hypéricacées: millepertuis, Iridacées; Juglandacées; Lamiacées: thym, origan, monarde, sarriette, basilic, marjolaines, menthes, patchouli, lavandes, sauges, cataire, romarin, hysope, mélisse; Lauracées: ravensara, laurier, bois de rose, cannelle, litséa; Liliacées: ail, lys, muguet, jacinthe, jonquille,...; Magnoliacées: magnolia; Malvacées ; Méliacées; Monimiacées; Moracées: chanvre, houblon; Myricacées; Mysristicacées: muscade; Myrtacées: eucalyptus, tea tree, niaouli, cajeput, backousia, girofle, myrte; Oléacées; Pipéracées: poivre ; Pittosporacées; Poacées: citronnelle, lemongrass, vétiver; Polygonacées; Renonculacées; Rosacées: roses; Rubiacées; Rutacées: tous les citrus; Salicacées; Santalacées: santal; Saxifragacées; Schisandracées; Styracacées: benjoin; Thymélacées: bois d'agar; Tilliacées; Valérianacées: valériane, nard; Verbénacées: lantana, verveine; Violacées; Zingibéracées: galanga, curcuma, cardamome, gingembre; Zygophyllacées.

On peut citer également les huiles essentielles extraites de fleurs (lys, lavande, rose, jasmin, Ylang-Ylang, néroli), de tiges et de feuilles (patchouli, géranium, petit-grain), de fruits (framboise, pêche , coriandre, anis, cumin, genièvre), d'écorces de fruits (bergamote, citron, orange, pamplemousse), de racines (angélique, céleri, cardamome, iris, acore, gingembre), de bois (bois de pin, santal, gaïac, cèdre rose, camphre), d'herbes et de graminées (estragon, romarin, basilic, lemon grass, sauge, thym), d'aiguilles et de branches (épicéa, sapin, pin, pin nain), de résines et de baumes ( galbanum, élémi, benjoin, myrrhe, oliban, opopanax).

Les exemples de substances parfumantes sont notamment : le géraniol, l'acétate de géranyle, le farnésol, le bornéol, l'acétate de bornyle, le linalool, l'acétate de linalyle, le propionate de linalyle, le butyrate de linalyle, le tétrahydrolinalool, le citronellol, l'acétate de citronellyle, le formiate de citronellyle, le propionate de citronellyle, le dihydromyrcénol, l'acétate de dihydromyrcényle, le tétrahydromyrcénol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzylcarbinol, l'acétate de trichlorométhylphénylcarbinyle, l'acétate de p-tert-butylcyclohexyle, l'acétate d'isononyle, l'acétate de cis-3-hexenyle , l'acétate de vétivéryle, l'acétate d'éthyle, l'acétate de butyle, l'acétate d'hexyle, l'acétate de décyle, l'acétate d'isoamyle, l'acétate de stéaryle, l'heptanoate d'allyle, le vétivérol, l'alpha-hexylcinnamaldéhyde, le 2-méthyl-3-(p-tert-butylphényl)propanal, le 2-méthyl-3-(p-isopropylphényl)propanal, le 3-(p-tert-butylphényl)-propanal, le 2,4-diméthylcyclohex-3-enyl-carboxaldéhyde, l'acétate de tricyclodécènyle, le propionate de tricyclodécènyle, l'allyl 3-cyclohexylpropionate, l'éthyl-6-(acétyloxy)-hexanoate, le caproate d'allyle, l'éthyl-2 méthyl butyrate, le méthyl dihydrojasmonate, le salicylate d'hexyle, le 4-(4-hydroxy-4-méthylpentyl)-3-cyclohexènecarboxaldéhyde, le 4-(4-méthyl-3-pentènyl)-3-cyclohexènecarboxaldéhyde, le 4-acétoxy-3-pentyl-tétrahydropyrane, le 3-carboxyméthyl-2-pentylcyclopentane, la 2-n-4-heptylcyclopentanone, la 3-méthyl-2-pentyl-2-cyclopentènone, la menthone, la carvone, la tagétone, la géranyl acétone, le n-décanal, le n-dodécanal, l'anisyl propanal, le 9-décènol-1, le cis-3-hexénol, le tétrahydro-2-isobutyl-4-méthylpyrann-4-ol, 3-Methyl-5-phényl-1-pentanol, le 3a,6,6,9a-Tétraméthyl-dodécahydronaphtho[2,1-b]furanne, l'isobutyrate de phénoxyéthyle, le phényl-acétaldéhyde diméthyl-acétal, le phénylacétaldéhyde diéthylacétal, le géranonitrile, le citronellonitrile, l'acétate de cédryle, le 3-isocamphylcyclohexanol, le cédryl méthyl éther, l'isolongifolanone, l'aubépinonitrile, l'aubépine, l'héliotropine, la coumarine, l'eugénol, la vanilline, l'oxyde de diphényle, le citral, le citronellal, l'hydroxycitronellal, l'hexylcinnamal, le 2,4-dimethylcyclohex-3-ène-1-carbaldéhyde, le 2,6-dimethylhept-5-ènal, l'$\alpha,\alpha$-diméthyl-p-éthylphénylpropanal, le 1,3-benzodioxole-5-carboxaldéhyde, le limonène, la damascone, la décalactone, la nonalactone, le 6,6-diméthoxy-2,5,5-triméthylhex-2-ène, la 2,4,4,7-tétraméthyloct-6-èn-3-one, la1-(5,5-diméthyl-1-cyclohexenyl)-pent-4-èn-1-one, la méthylheptènone, le 4-(cyclopropylméthyl)-phénylméthyl éther, le 2-methyl-6-methylideneoct-7-en-2-ol, l'oxyde de rose, la 1-(1,2,3,4,5,6,7,8-octahydro-2,3,8,8-tetraméethyl-2-naphthyl)éthan-1-one, la 2-acétonaphthone, la 2-isopropyl-5-méthylcyclohexanone, les ionones, les méthylionones, les isométhylionones, la solanone, les irones, le cis-3-hexénol et ses esters, les muscs-indanes, les muscs-tétralines, les muscs-isochromanes, les cétones macrocycliques, les muscs-macrolactones, les muscs aliphatiques, le brassylate d'éthylène, l'essence de rose et leurs mélanges.

D'une façon générale, les parfums sont constitués d'un mélange d'ingrédients dits de parfumerie qui peuvent être également classés en notes de tête, notes de cœur et notes de fond.

Les trois notes correspondent à la volatilité plus ou moins importante des ingrédients qui les composent: note de tête fortement volatile, note de coeur moyennement volatile et note de fond faiblement volatile.

(i) La note de tête appelée aussi « départ » est celle que l'odorat perçoit en premier dès que le parfum est en contact avec la matière kératinique ou tout substrat. Mais c'est celle qui s'atténue le plus rapidement: elle ne "tient pas". Il est difficile d'exprimer le temps de persistance de cette note, car il est très variable: de quelques minutes à une dizaine de minutes

Elle est essentiellement fraîche et légère. Tous les agrumes appartiennent notamment à cette catégorie. En parfumerie, on les range sous le terme générique d'hespéridés dont font partie orange, citron, pamplemousse, bergamote, néroli etc... On citera également les aromates tels que la lavande, le laurier, le thym ou le romarin et les anisés, mentholés, aldéhydés, etc. On citera également les notes eucalyptus.

(ii) La note de cœur, parfois appelée aussi «corps» a une persistance qui va de quelques dizaines de minutes à quelques heures, mais sa principale caractéristique est de ne se révéler qu'au bout de quelques minutes. Elle "démarre" donc juste avant l'extinction de la note de tête. Elle commence à s'exprimer alors que la note de tête s'efface progressivement. Elle est représentée essentiellement par des éléments floraux, fruités ou épicés: muguet, chèvrefeuille, violette, magnolia, cannelle, géranium, jasmin, rose, iris, framboise, pêche, etc...

(ii) La note de fond, parfois appelée aussi «fond» assure la "durabilité", la persistance ou la ténacité d'un parfum. Elle est perceptible plusieurs heures, voire plusieurs jours, ou même plusieurs semaines après application. sur un vêtement ou sur une mouillette ou touche olfactive, selon la concentration du parfum. On citera par exemple les bois, racines, mousses, résines et les substances animales ou minérales telles que opoponax, muscs, ambre, santal, benjoin, lichen, clou de girofle, sauge, etc. On citera également les notes vanillées, le patchouli, les coumarines...etc.

On peut bien entendu encapsuler des ingrédients appartenant à une ou plusieurs notes. Toutefois, on préférera encapsuler les ingrédients les plus volatiles (= les moins rémanents) appartenant aux notes de tête et/ou de cœur. Parmi ces ingrédients, on citera, par exemple :

Acétate de benzyle
Acétate de géranyle
Acétate de cis-3-hexenyle
Aldéhyde $C_{18}$ ou nonalactone
Acétate de décyle
Allyl amyl glycolate (citral)
Acétate d'éthyle
Acétate de butyle
Allyl 3-cyclohexylpropionate
Acétate de linalyle
Alcool Phényléthylique
Acétate d'héxyle
Berryflor ou éthyl-6-(acétyloxy)-hexanoate
Acétate d'isoamyle
Caproate d'allyle
Amarocite ou 6,6-diméthoxy-2,5,5-triméthylhex-2-ène
Citral lemarome N ou 3,7-diméthylocta-2,6-diénal
Canthoxal ou anisyl propanal
Claritone ou 2,4,4,7-tétraméthyloct-6-èn-3-one
Ethyl-2méthyl butyrate
Dihydromyrcénol
Cis-3 hexenol
Hédione ou méthyl dihydrojasmonate
L-carvone
Heptanoate d'allyle
Limonène
Néobuténone alpha ou 1-(5,5-diméthyl-1-cyclohexenyl)- pent-4-èn-1-one
Méthylheptènone
Toscanol ou 4-(cyclopropylméthyl)-phénylméthyl éther
myrcenol super ou 2-methyl-6-methylideneoct-7-en-2-ol
Decalactone
Acétate de stéaryle
Oxyde de rose
Linalool
Triplai ou 2,4-dimethylcyclohex-3-ène-1-carbaldéhyde
Mélonal ou 2,6-dimethylhept-5-ènal
1-(1,2,3,4,5,6,7,8-octahydro-2,3,8,8-tetraméethyl-2-naphthyl)éthan-1-one
Hexylcinnamal

Tétrahydro-2-isobutyl-4-méthylpyrann-4-ol
Salicylate d'hexyle
1,4-Dioxacycloheptadécane-5,17-dione
et leurs mélanges

Selon une forme particulière de l'invention, les particules d'encapsulation comprennent au moins une ou plusieurs substances parfumantes ayant une pression de vapeur saturante à 25°C supérieure ou égale à 10,0Pa

La pression de vapeur saturante (ou tension de vapeur) est la pression à laquelle la phase gazeuse d'une substance est en équilibre avec sa phase liquide ou solide à une température donnée dans un système fermé. Le calcul de la pression de vapeur saturante peut se faire à l'aide de la formule suivante :

$$\ln \frac{p_{\text{sat}}}{p_0} = \frac{M.L_v}{R}\left(\frac{1}{T_0} - \frac{1}{T}\right)$$

avec :

- $T_0$ : température d'ébullition de la substance à une pression $p_0$ donnée, en degrés Kelvin,
- $p_{\text{sat}}$ : pression de vapeur saturante, dans la même unité que $p_0$
- M : masse molaire de la substance, en kg/mol
- $L_v$ : chaleur latente de vaporisation de la substance, en Joules/kg
- R : constante des gaz parfaits, égale à 8,31447J/K/mol
- T : température de la vapeur, en K.

De préférence les substances parfumantes ayant une pression de vapeur saturante à 25°C supérieure ou égale à 10Pa représentent une quantité allant de 50 à 100% en poids, de préférence de 60 à 100% en poids, plus préférentiellement de 70 à 100% en poids, et encore mieux de 80 à 100% en poids par rapport au poids total des substances parfumantes présentes dans les particules de l'invention.

### c) Les principes actifs pharmaceutiques

On entend par «principe actif pharmaceutique» une molécule ou un mélange de molécules qui possède un effet thérapeutique, curatif et/ou prophylactique pouvant être administré par pulvérisation.

### d) Les actifs cosmétiques

On entend par «actif cosmétique» toute molécule qui possède un effet d'hygiène, de soin, de maquillage, de coloration contribuant à l'amélioration, du bien-être et/ou à l'embellissement ou la modification de l'aspect de la matière kératinique humaine sur laquelle on applique ladite composition.

Parmi les actifs cosmétiques susceptibles d'être appliqués sur des matières kératiniques humaines telles que la peau, les lèvres, le cuir chevelu, les cheveux, les cils ou les ongles, on peut citer par exemple, seuls ou en mélanges

- les vitamines et leurs dérivés ou précurseurs, seuls ou en mélanges,
- les agents anti-oxydants
- les agents nettoyants tels que les tensio-actifs
- les matières colorantes
- les agents conditionneurs
- les agents pour le défrisage et/ou le lissage et/ou la mise en forme des cheveux
- les agents anti-radicalaires
- les agents photoprotecteurs comme les filtres UV organiques ou inorganiques
- les agents autobronzants
- les agents anti-glycation
- les agents apaisants
- les agents dépilatoires
- les agents déodorants
- les agents anti-transpirants
- les inhibiteurs de NO-synthase

- les agents stimulant la prolifération des fibroblastes
- les agents stimulant la prolifération des kératinocytes
- les agents dermorelaxants
- les agents rafraîchissants
- les agents tenseurs
- les agents matifiants
- les agents anti-luisance de la peau
- les agents anti-séborrhéiques
- les agents anti-cheveux gras
- les agents dépigmentants
- les agents pro-pigmentants
- les agents kératolytiques
- les agents desquamants
- les agents hydratants
- les agents anti-microbiens
- les agents amincissants
- les agents agissant sur le métabolisme énergétique des cellules
- les agents répulsifs contre les insectes
- les antagonistes de substances P ou de CRGP
- les agents anti-chute des cheveux
- les agents anti-rides
- les agents anti-vieillissement
- les agents antipelliculaires

Parmi ces actifs cosmétiques, on préférera tout particulièrement, seuls ou en mélanges

- les agents photoprotecteurs comme les filtres UV en particulier les filtres UV organiques
- les agents anti-luisance de la peau
- les agents anti-séborrhéiques
- les agents anti-cheveux gras
- les agents déodorants
- les agents anti-transpirants
- les agents rafraîchissants
- les agents matifiants
- les agents anti-microbiens
- les agents antipelliculaires
- les agents anti-oxydants

Selon une forme particulièrement préférée de l'invention, le ou les agents bénéfiques présents dans les particules seront choisis parmi les substances parfumantes.

Selon une forme encore plus particulièrement préférée de l'invention, les substances parfumantes présentes dans les particules sont choisies parmi les notes de cœur et/ou les notes de tête de façon à pouvoir aussi bien compenser leur perte tout au long de la journée qu'à procurer un effet fraicheur supplémentaire au cours de la journée en réponse à la transpiration comme à l'humidité atmosphérique ou apportée par exemple par des brumisateurs.

Selon une forme particulière de l'invention, on choisira de préférence les substances parfumantes ayant une pression de vapeur saturante à 25°C supérieure ou égale à 10,0Pa

Selon une forme particulière de l'invention, la composition contiendra

a) :des particules renfermant au moins une substance parfumante et

b) au moins une substance parfumante sous forme libre, identique ou différente de la substance parfumante présente dans les dites particules.

Lesdites substances parfumantes sous forme libre peuvent être choisies parmi celles citées précédemment.

Selon une autre forme particulière de l'invention, la composition contient exclusivement la ou les substances parfumantes dans les particules d'encapsulation. Autrement dit, la totalité des ingrédients pour parfumer présents dans la composition sont contenus dans les particules.

La composition peut comprendre, en outre, d'autres ingrédients sous forme libre (non encapsulés, emprisonnés dans les particules de l'invention) utilisés couramment dans les compositions cosmétiques. De tels ingrédients peuvent être choisis parmi les antioxydants, les conservateurs, les actifs cosmétiques tels que ceux cités précédemment, les substances parfumantes telles que celles décrites précédemment et leurs mélanges.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Les compositions selon l'invention peuvent se présenter sous toute forme acceptable et usuelle pour une composition cosmétique ou dermatologique sous forme de poudre libre ou compacte.

L'homme du métier pourra choisir la composition appropriée, ainsi que sa méthode de préparation, sur la base de ses connaissances générales, en tenant compte d'une part de la nature des constituants utilisés et d'autre part de l'application envisagée pour la composition.

Selon une forme particulière de l'invention, les compositions selon l'invention sont des produits de maquillage comme des poudres pour le teint, des fards à joue, des fards à paupières où la composition comprend au moins un agent de coloration sous forme libre (non encapsulé) et/ou sous forme encapsulée notamment choisi parmi las nacres, les pigments, les particules réfléchissantes, et leur(s) mélange(s). Plus particulièrement, les particules selon l'invention comprennent au moins une substance parfumante. Encore plus particulièrement, les compositions contiendront en plus une substance parfumante sous forme libre, identique ou différente de la substance parfumante présente dans les particules.

Selon une autre forme particulière de l'invention, les compositions selon l'invention peuvent se présenter sous forme de produits capillaires. Ces produits capillaires peuvent être notamment des produits destinés au nettoyage non rincé des cheveux comme des «shampooings secs», des produits pour le soin et/ou le traitement cosmétique des cheveux et du cuir chevelu. Plus particulièrement, les particules comprennent au moins une substance parfumante. Encore plus particulièrement, les compositions contiendront en plus une substance parfumante sous forme libre, identique ou différente de la substance parfumante présente dans les particules.

Selon une autre forme particulière de l'invention, les compositions selon l'invention peuvent se présenter sous forme de produit d'hygiène, en particulier des déodorants et/ou anti-transpirants où la composition comprend au moins un actif déodorant et/ou au moins un actif anti-transpirant, sous forme libre et/ou sous forme encapsulée. Plus particulièrement, les particules comprennent au moins une substance parfumante. Encore plus particulièrement, les compositions contiendront en plus au moins une substance parfumante sous forme libre, identique ou différente de la ou des substances parfumantes présentes dans les particules.

## Actif anti-transpirant

Par «actif anti-transpirant», on entend un composé qui, à lui seul, a pour effet de diminuer le flux sudoral, et/ou de diminuer la sensation d'humidité sur la peau liée à la sueur humaine et/ou d'absorber partiellement ou totalement la sueur humaine.

Parmi les actifs anti-transpirants, on peut citer les sels d'aluminium et/ou de zirconium tels que le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate et plus particulièrement le chlorhydrate d'aluminium sous forme activée ou non commercialisé par la société REHEIS sous la dénomination MICRODRY ALUMINUM CHLOROHYDRATE® ou par la société GUILINI CHEMIE sous la dénomination ALOXICOLL PF 40. Des sels d'aluminium et de zirconium sont par exemple celui commercialisé par la société REHEIS sous la dénomination REACH AZP-908-SUF®, des sels d'aluminium « activés » par exemple celui commercialisé par la société REHEIS sous la dénomination REACH 103 ou par la société WESTWOOD sous la dénomination WESTCHLOR 200.

De préférence, la composition cosmétique comprend le chlorhydrate d'aluminium en tant qu'actif anti-transpirant.

Comme autre actif anti-transpirant, on peut citer les particules de perlite expansée telles que celles obtenues par le procédé d'expansion décrit dans le brevet US 5,002,698.

Les perlites utilisables selon l'invention sont généralement des aluminosilicates d'origine volcanique et ont comme composition

70,0-75,0% en poids de silice $SiO_2$

12,0-15,0% en poids d'oxyde d'aluminium oxyde $Al_2O_3$

3,0-5,0% d'oxyde de sodium $Na_2O$

3,0-5,0% d'oxyde de potassium $K_2O$

0,5-2% d'oxyde de fer $Fe_2O_3$

0,2-0,7% d'oxyde de magnésium MgO
0,5-1,5% d'oxyde de calcium CaO
0,05-0,15% d'oxyde de titane $TiO_2$

De préférence, les particules de perlite utilisées seront broyées; elles sont dans ce cas dites Expanded Milled Perlite (EMP). Elles ont de préférence une taille de particule définie par un diamètre médian D50 allant de 0,5 à 50 $\mu$m et de préférence de 0,5 à 40 $\mu$m.

De préférence, les particules de perlite utilisées présentent une densité apparente non tassée à 25°C allant de 10 et 400 kg/m3 (Norme DIN 53468) et de préférence de 10 et 300 kg/m3.

De préférence, les particules de perlite expansée selon l'invention ont une capacité d'absorption d'eau mesurée au WET POINT allant de 200 à 1500% et de préférence de 250 à 800%.

Le Wet Point correspond à la quantité d'eau qu'il faut additionner à 100 g de particule pour obtenir une pâte homogène. Cette méthode dérive directement de celle de la prise d'huile appliquée aux solvants. Les mesures sont faites de la même manière par l'intermédiaire du Wet Point et du Flow Point ayant respectivement comme définition suivante :

WET POINT: masse exprimée en grammes pour 100g de produit correspondant à l'obtention d'une pâte homogène lors de l'addition d'un solvant à une poudre.

FLOW POINT: masse exprimée en grammes pour 100g de produit à partir de laquelle la quantité de solvant est supérieure à la capacité de la poudre à le retenir. Cela se traduit par l'obtention d'un mélange plus ou moins homogène s'écoulant sur la plaque de verre.

Le Wet Point et le Flow point sont mesurés selon le protocole suivant:

Protocole de mesure de l'absorption d'eau.

1) Matériel utilisé

Plaque de verre (25 x 25 mm)
Spatule (manche en bois et partie métallique (15 x 2,7mm)
Pinceau à poils de soie
Balance

2) Mode Opératoire

On dépose la plaque de verre sur la balance et on pèse 1g de particules de perlite. On dépose le bécher contenant le solvant ainsi que la liquipipette de prélèvement sur la balance. On ajoute progressivement le solvant à la poudre en malaxant régulièrement l'ensemble (toutes les 3 à 4 gouttes) à l'aide de la spatule

On note la masse de solvant nécessaire à l'obtention du Wet Point. On ajoute à nouveau le solvant et on note la masse permettant d'arriver au Flow Point. On effectuera la moyenne sur 3 essais.

On utilisera en particulier les particules de perlite expansée vendues sous les noms commerciaux OPTIMAT 1430 OR ou OPTIMAT 2550 par la société WORLD MINERALS.

**Actifs déodorants**

On appelle « actif déodorant », toute substance capable de masquer, absorber, améliorer et/ou réduire l'odeur désagréable résultant de la décomposition de la sueur humaine par des bactéries

Les actifs déodorants peuvent être des agents bactériostatiques ou des agents bactéricides agissant sur les germes des odeurs axillaires, comme le 2,4,4'-trichloro-2'-hydroxydiphényléther (®Triclosan), le 2,4-dichloro-2'-hydroxydiphényléther, le 3',4',5'-trichlorosalicylanilide, la 1-(3',4'-dichlorophenyl)-3-(4'-chlorophenyl)urée (®Triclocarban) ou le 3,7,11-triméthyldodéca-2,5,10-triénol (®Farnesol) ; les sels d'ammonium quaternaires comme les sels de cetyltrimethylammonium, les sels de cétylpyridinium ; les polyols comme ceux de type glycérine, 1,3-propanediol (ZEMEA PROPANEDIOL® commercialisé par Dupont Tate and Lyle Bioproducts), le 1,2-décanediol (SYMCLARIOL® de la société Symrise) ; les dérivés de glycérine comme par exemple le Caprylic/Capric Glycerides (CAPMUL MCM® de Abitec), le Caprylate ou caprate de Glycerol (DERMOSOFT GMCY® et DERMOSOFT GMC® respectivement de STRAETMANS), le Polyglyceryl-2 Caprate (DERMOSOFT DGMC® de STRAETMANS) les dérivés de biguanide comme les sels de polyhexaméthylène biguanide ; la chlorhexidine et ses sels; le 4-Phenyl-4,4-dimethyl-2butanol (SYMDEO MPP® de Symrise) ; les cyclodextrines ; les chélatants tels que le Tetrasodium Glutamate Diacetate (CAS #51981-21-6) vendu sous le nom commercial DISSOLVINE GL-47-S® de Akzo Nobel, l'EDTA (acide Ethylendiamino Tétraacétique) et le DPTA (acide 1,3-diaminopropanetétraacétique).

Parmi les actifs déodorants conformes à l'invention, on peut aussi citer également

- les sels de zinc comme le salicylate de zinc, le phénolsulfonate de zinc, le pyrrolidone carboxylate de zinc (plus communément appelé pidolate de zinc), le sulfate de zinc, le chlorure de zinc, le lactate de zinc, le gluconate de zinc, ricinoléate de zinc, glycinate de zinc, carbonate de zinc, citrate de zinc, chlorure de zinc, le laurate de zinc, l'oléate de zinc, l'orthophosphate de zinc, le stéréate de zinc, le tartrate de zinc, l'acétate de zinc ou leurs mélanges ;
- des absorbeurs d'odeurs comme les zéolites notamment métalliques sans argent, les cyclodextrines, les silicates d'oxyde métallique telles que celles décrite dans la demande US2005/063928 ; des particules d'oxyde métallique modifiées par un métal de transition telles que décrites dans les demandes US2005084464 et US2005084474, des aluminosilicates comme ceux décrits dans la demande EP1658863, des particules de dérivés de chitosan comme celles décrites dans le brevet US6916465 ;
- le bicarbonate de sodium ;
- l'acide salicylique et ses dérivés tels que l'acide n-octanoyl-5-salicylique
- l'alun.
- le triéthyl citrate

Les actifs déodorants peuvent être présents de préférence dans les compositions selon l'invention dans des concentrations pondérales allant 0,01 à 10% en poids par rapport au poids total de la composition.

La présente invention concerne également un procédé cosmétique de traitement des odeurs corporelles et éventuellement de la transpiration humaine consistant à appliquer sur la matière kératinique une composition comprenant les particules telles que définies précédemment ; ladite composition comprenant au moins un actif déodorant et/ou au moins un actif anti-transpirant, sous forme libre et/ou sous forme encapsulée.

La composition sous forme de poudre compacte peut être préparée en mélangeant les ingrédients de la phase pulvérulentes (charges et pigments) puis en ajoutant la phase grasse sous agitation, le mélange étant ensuite broyé, puis en ajoutant les capsules de parfum, le mélange étant ensuite tamisé, puis versé dans une coupelle et compacté.

La poudre est compactée à l'aide d'une presse, notamment en appliquant une pression allant de 50 à 150 bars $(5 \times 10^6 \text{ à } 15 \times 10^6 \text{ Pa})$.

La phase grasse de la poudre compacte, appelée généralement liant, est une phase grasse liquide à la température ambiante (25 °C) et peut comprendre une huile utilisée habituellement dans les poudres compactes.

L'huile peut être choisie parmi les huiles utilisées classiquement comme liant dans les poudres compactes. Parmi les huiles additionnelles utilisables, on peut citer l'huile de vison, l'huile de tortue, l'huile de soja, l'huile de pépins de raison, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide; les huiles d'hydrocarbures, telles que les huiles de paraffine, le squalane, la vaseline, le polydécène ; les esters gras, tels que le myristate d'isopropyle, le palmitate d'isopropyle, le stéarate de butyle, le stéarate d'isodécyle, le laurate d'hexyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine; les huiles de silicone telles que les polyméthylsiloxanes, les polyméthylphénylsiloxanes, les polysiloxanes modifiés par des acides gras, des alcools gras ou des polyoxyalkylènes, les silicones fluorées, les huiles perfluorées; l'acide oléique, l'acide linoléique, l'acide linolénique ; les alcools gras supérieurs tels que le cétanol ou l'alcool oléique.

La phase grasse liquide peut être présente en une teneur allant de 0,1 % à 20 % en poids, par rapport au poids total de la composition, de préférence allant de 0,1 % à 15 % en poids, et préférentiellement allant de 0,1 % à 10 % en poids.

L'invention est illustrée plus en détail dans les exemples suivants.

## Exemples de préparation de particules à libération de parfum

### Exemple A

On a préparé des capsules en mettant en œuvre la composition suivante :

| Composition | Amidon modifié hydrophobe | Polysaccharide hydrosoluble | Parfum* | Eau |
|---|---|---|---|---|
| **Exemple A** | Amidon Capsul® de National Starch 110g | Maltodextrine de pomme de terre MD 20 P de AVEBE 110g | 55g | 225 g |

\* Le parfum utilisé a la composition suivante :

| Ingrédients | Quantité en g |
|---|---|
| Myristate d'isopropyle | 20,5 |
| Méthyl dihydrojasmonate | 15 |
| 2-phenyléthanol | 8 |
| 1-(1,2,3,4,5,6,7,8-octahydro-2,3,8,8-tetraméthyl-2-naphthyl)éthan-1-one | 8 |
| Hexylcinnamal | 6 |
| Tétrahydro-2-isobutyl-4-méthylpyrann-4-ol | 6 |
| Hexyl Salicylate | 6 |

| | |
|---|---|
| Benzyl Acétate | 5 |
| 1,4-Dioxacycloheptadécane-5,17-dione | 5 |
| 3-Methyl-5-phényl-1-pentanol | 5 |
| dihydromyrcenol | 4 |
| ORANGE TERPENES 0,05% B H T (limonène >95%) | 4 |
| 2-acétonaphthone | 2 |
| 3a,6,6,9a-Tétraméthyl-dodécahydronaphtho[2,1-b]furanne | 1 |
| α,α-diméthyl-p-éthylphénylpropanal | 1 |
| 1,3-benzodioxole-5-carboxaldéhyde | 1 |
| 2-isopropyl-5-méthylcyclohexanone | 1 |
| 1-phényléthyl acetate | 0,8 |
| 2,6-dimethylhept-5-ènal (mélonal) | 0,5 |
| 2,4-dimethylcyclohex-3-ène-1-carbaldehyde (triplal) | 0,2 |

**Procédé de préparation de l'émulsion**

On a mélangé la maltodextrine de pomme de terre MD20 P et l'amidon CAPSUL® (sel de sodium de l'octényle succinate d'amidon) dans l'eau jusqu'à dissolution puis on a ajouté le parfum et émulsionné avec un disperseur Ultraturrax de type Heidolph Diax 900 (moteur de puissance 900W avec une vitesse de 8000 à 26000 tour/mn électroniquement contrôlée) à la puissance maximale pendant 4 minutes

**Procédure de séchage pour obtenir des particules sphériques**

L'émulsion obtenue a été ensuite homogénéisée à une pression de 30 bars au moyen d'une pompe à haute pression puis pulvérisée dans une chambre d'atomisation au moyen d'une buse simultanément avec un courant de $CO_2$ (30 bars, 45°C) que l'on a fait circuler en continu à un débit d'environ 500g/mn pour éliminer l'eau. La poudre séchée a été retenue sur un filtre situé à la base de la chambre d'atomisation puis collectée après dépressurisation. On obtient ainsi 270g de microcapsules sphériques sous la forme d'une fine poudre blanche ayant un diamètre moyen en nombre de 7,8 $\mu$m et un diamètre moyen en volume de 47 $\mu$m.

La taille des particules a été mesurée en voie sèche par diffraction laser en utilisant un granulomètre Microtrac S3500, les granulométries étant exprimées en volume et en nombre.

| Exemple A | Caractéristiques mesurées des capsules | | | |
|---|---|---|---|---|
| | Taux de parfum encapsulé (%) | Taux de parfum libre (%) | Densité de poudre versée | Densité absolue |
| | 19,8 | < 0,1 | 484 | 1,12 |

**Exemples B à H**

Selon le procédé décrit à l'exemple A, on a préparé les capsules suivantes :

| Composition | Amidon modifié hydrophobe | Polysaccharide hydrosoluble | Parfum de l'exemple A | Eau |
|---|---|---|---|---|
| Exemple B | Amidon Capsul® de National Starch 110g | Maltodextrine MD 120 de Tereos  110g | 55g | 225 g |
| Exemple C | Amidon Capsul® de National Starch 110g | Maltodextrine MD 170 de Tereos  110g | 55g | 225 g |
| Exemple D | Amidon Capsul® de National Starch 110g | Maltodextrine MD 190 de Tereos  110g | 55g | 225 g |

| Exemple E | Amidon Capsul® de National Starch 110g | Maltodextrine de pomme de terre MD 20 P de AVEBE 110g | 105g | 225 g |
|---|---|---|---|---|

| Exemple F | Amidon Capsul® de National Starch 154g | Maltodextrine de pomme de terre MD 20 P de AVEBE 66g | 55g | 22 5g |
|---|---|---|---|---|
| Exemple G | Amidon Capsul® de National Starch 66g | Maltodextrine de pomme de terre MD 20 P de AVEBE 154g | 55g | 22 5g |
| Exemple H | Amidon Capsul® de National Starch 110g | Sirop de glucose Glucodry G290 de TEREOS 110g | 55g | 22 5g |

| Exemples | Caractéristiques mesurées des capsules | | | |
|---|---|---|---|---|
| | Taux de parfum encapsulé (%) | Taux de parfum libre (%) | Densité de poudre versée (g/l) | Densité absolue |
| Exemple B | 19,3 | <0,1 | 568 | 1,14 |
| Exemple C | 19,4 | <0,1 | 490 | 1,16 |
| Exemple D | 19,9 | <0,1 | 537 | 1,11 |
| Exemple E | 38 | 0,8 | 482 | 1,08 |
| Exemple F | 21,0 | 0,2 | 595 | 1,11 |
| Exemple G | 20,7 | 0,2 | 521 | 1,15 |
| Exemple H | 19,2 | 0,1 | 568 | 1,12 |

**Exemple I comparatif**

On a réalisé des capsules ayant la même composition que l'exemple A tel décrit ci-dessus selon le procédé de l'exemple 1 du brevet US6200949 comprenant un séchage par spray-drying (atomisation) de l'émulsion.

L'émulsion est séchée par spray-drying au moyen d'un appareil du type Bowen Lab Model Dryer utilisant de l'air avec un débit de 420m$^3$/h à une température de 204°C et une température externe de 93°C et une vitesse de la turbine de 50000 tr/mn.

Aspect morphologique des particules obtenues: polymorphe avec agrégats

**Exemple J comparatif**

On a réalisé des capsules ayant la même composition que l'exemple A tel décrit ci-dessus selon le procédé de l'exemple 1 du du brevet US5508259 comprenant un séchage par spray-drying (atomisation) de l'émulsion.

Le mélange a été séché par spray-drying avec un appareil du type CCM Sulzer avec un débit d'émulsion de 50kg/h, de l'air à un débit de 320m$^3$/h à 350 °C et 0,45 bar.

Aspect morphologique des particules obtenues : polymorphe avec agrégats

| Composition | Caractéristiques mesurées des capsules | | | |
|---|---|---|---|---|
| | Taux de parfum encapsulé (%) | Taux de parfum libre (%) | Densité de poudre versée (g/l) | Densité absolue |
| Exemple I (hors invention) | 18,3 | 2,7 | 259 | 1,16 |
| Exemple J (hors invention) | 11,2 | 1,7 | 269 | 1,12 |

**Exemple 1 : Préparation d'une poudre libre déodorante ayant la composition suivante :**

| Ingrédients | (% en poids) |
|---|---|
| Talc | 34,4 |
| Amidon de maïs | 30 |
| Perlite | 30 |
| Capsules de parfum A | 5,0 |
| Acide salicylique | 0,1 |
| Octane-1.2-diol | 0,5 |
| Total | 100,00 |

On a introduit 17,2g de talc, 15,0g d'amidon de maïs, 15,0g de perlite et 0,05g d'acide salicylique dans une cuve équipée d'un agitateur. On a homogénéisé le mélange sous agitation vive (1500tr/mn). Puis on a ajouté 2,5g de capsules en homogénéisant à nouveau le mélange. On a ajouté enfin 0,25g d'octanediol sous une agitation lente. Après avoir tamisé à 250 microns, on a obtenu une poudre de couleur blanc-beige.

**Exemples C1 et C2: Produits déodorants**

Similairement à l'exemple 1, on a préparé des poudres déodorantes ayant les compositions suivantes :

| Poudre C1 (hors invention) | |
|---|---|
| Ingrédients | (% en poids) |
| Talc | 34,4 |
| Amidon de maïs | 30 |
| Perlite | 30 |
| Capsules de l'exemple I | 5,0 |
| Acide salicylique | 0,1 |
| Octane-1.2-diol | 0,5 |
| Total | 100,00 |

| Poudre C2 (hors invention) | |
| --- | --- |
| **Ingrédients** | **(% en poids)** |
| Talc | 34,4 |
| Amidon de maïs | 30 |
| Perlite | 30 |
| Capsules de l'exemple J | 5,0 |
| Acide salicylique | 0,1 |
| Octane-1.2-diol | 0,5 |
| Total | 100,00 |

**Comparaison des formules Exemple 1, Poudres C1 et C2** :

**Protocole d'évaluation** :

On pèse 0,1g de composition dans une boite de Pétri. Après une minute, on évalue l'intensité olfactive d'échantillon qu'on note de 0 à 10. Puis on simule la transpiration par un ajout d'eau d'environ 0,1g (2 sprays) sur la composition déposée. On attend 30 secondes et évalue l'intensité olfactive.

| Formule | Aspect | Toucher | Intensité olfactive | |
| --- | --- | --- | --- | --- |
| | | | **Avant Ajout d'eau** | **Après 2 sprays d'eau** |
| **Exemple 1 (invention)** | Poudre libre homogène | Doux Lisse | 0 | 8 |
| **Poudre C1** | Poudre hétérogène | Granuleux | 6 | 8 |
| **Poudre C2** | Poudre hétérogène | Granuleux | 5 | 7 |

Echelle d'intensité odeur : 0 à 10 (0 inodore ; 10 odeur très intense / saturée).

On observe que la poudre de l'invention (exemple 1) est inodore avant ajout d'eau contrairement aux poudres C1 et C2, (hors invention), ce qui confirme l'étanchéité des capsules alors que les capsules de parfum dans les poudres C1 et C2 ne sont pas étanches avant même l'ajout d'eau

**Exemple 2: Préparation d'une poudre libre corporelle avant la composition suivante :**

| Ingrédients | (% en poids) |
| --- | --- |
| Talc | 76,2 |
| MAGNESIUM CARBONATE | 0,7 |
| MICA (and) TITANIUM DIOXIDE | 4,0 |
| Capsules de parfum A | 13,6 |
| MICA (and) TITANIUM DIOXIDE (and) IRON OXIDES | 3,0 |
| Parfum libre | 1,0 |
| ACRYLATES COPOLYMER (EXPANCEL 551 DE 40 D42 ®) | 1,5 |
| Total | 100,00 |

Les capsules de l'exemple A peuvent être remplacées par les capsules des exemples B à H décrites précédemment.

On a introduit 15,2g de talc, 0,14g de carbonate de magnésium, 0,8g de « mica (and) dioxyde de titanium », 0,6g de « mica (and) dioxyde de titanium (and) oxyde de fer » et 0,3g de copolymère d'acrylate dans une cuve équipée d'un agitateur. On a homogénéisé le mélange sous agitation puis on a ajouté 0,2g de parfum libre et 2,7g de capsules en homogénéisant le mélange sous une agitation lente. Après on a passé le mélange à travers un tamis de 250 microns et on a obtenu ainsi une poudre beige-marron.

La composition appliquée sur le corps libère le parfum au cours de la journée au contact de la transpiration ou de l'humidité.

**Exemple** 3 : **Poudre libre corporelle**

De façon similaire à l'exemple 2, on a préparé la poudre corporelle suivante :

| Ingrédients | (% en poids) |
|---|---|
| Talc | 77,2 |
| MAGNESIUM CARBONATE | 0,7 |
| MICA (and) TITANIUM DIOXIDE | 4,0 |
| Capsules de parfum A | 13,6 |
| MICA (and) TITANIUM DIOXIDE (and) IRON OXIDES | 3,0 |
| ACRYLATES COPOL YMER (EXPANCEL 551 DE 40 D42 ®) | 1,5 |
| Total | 100,00 |

Les capsules de l'exemple A peuvent être remplacées par les capsules des exemples B à H décrites précédemment.

La composition appliquée sur le corps libère le parfum au cours de la journée au contact de la transpiration ou de l'humidité.

**Exemple 4: Préparation d'un shampoing sec (poudre libre) avant la composition suivante**

| Ingrédients | (% en poids) |
|---|---|
| Carbonate de calcium | 14,8 |
| Amidon de maïs estérifié par anhydride dodecylsuccinique, sel de calcium (DRY-FLO® AF PURE de chez Akzo Nobel) | 75,0 |
| Hectorite modifiée distearyl ammonium | 2,0 |
| Myristate d'isopropyle | 3,2 |
| Capsules de parfum A | 5,0 |
| Total | 100,00 |

Les capsules de l'exemple A peuvent être remplacées par les capsules des exemples B à H décrites précédemment.

On a introduit 14,8g de carbonate de calcium, 75,0g d'amidon de maïs, 2,0g d'hectorite modifiée et 3,2g de myristate d'isopropyle dans une cuve équipée d'un agitateur avec une turbine de fond. On a turbiné et agité pendant 3 min à 1500tr/mn. Puis on a ajouté 5,0g de capsules en homogénéisant à nouveau le mélange. On a obtenu après tamisage une poudre blanc-beige.

Après l'application de la composition sur les cheveux, on constate lorsque le sujet transpire ou au contact du sébum qu'une libération du parfum se produit au cours de la journée.

**Exemple 5 : Préparation d'un shampoing sec anhydre (poudre libre) avant la composition suivante** :

| Ingrédients | (% en poids) |
|---|---|
| Carbonate de calcium | 14,8 |
| Amidon de maïs estérifié par anhydride dodecylsuccinique, sel de calcium (DRY-FLO® AF PURE de chez Akzo Nobel) | 76,4 |
| Hectorite modifiée distearyl ammonium | 2,0 |
| Myristate d'isopropyle | 3,0 |
| Capsules de parfum A | 3,0 |
| Parfum libre | 0,8 |
| Total | 100,00 |

Les capsules de l'exemple A peuvent être remplacées par les capsules des exemples B à H décrites précédemment.

On introduit 14,8g de carbonate de calcium, 76,4g d'amidon de maïs, 2,0g d'hectorite modifiée, 3,0g de myristate d'isopropyle et 0,8g du parfum libre dans une cuve équipée d'un agitateur avec turbine de fond. On agite pendant 3 min à 1500tr/mn. Puis on ajoute 3,0g de capsules de l'exemple A en homogénéisant le mélange. On obtient ainsi, après tamisage, une poudre blanc-beige.

Après l'application de la composition sur les cheveux, on constate lorsque le sujet transpire ou au contact du sébum qu'une libération du parfum se produit au cours de la journée.

**Exemple 6 : Préparation d'un shampoing sec anhydre (poudre libre) avant la composition suivante**

| Ingrédients | (% en poids) |
|---|---|
| Carbonate de calcium | 14,8 |
| Amidon de maïs estérifié par anhydride dodecylsuccinique, sel de calcium (DRY-FLO® AF PURE de chez Akzo Nobel) | 76,4 |
| Hectorite modifiée distearyl ammonium | 2,0 |
| Myristate d'isopropyle | 3,0 |
| Capsules de parfum F | 3,0 |
| Parfum libre | 0,8 |
| Total | 100,00 |

Les capsules de l'exemple F peuvent être remplacées par les capsules des exemples A à E et G, H décrites précédemment.

On a introduit 14,8g de carbonate de calcium, 76,4g d'amidon de maïs, 2,0g d'hectorite modifiée, 3,0g de myristate d'isopropyle et 0,8g du parfum libre dans une cuve équipée d'un agitateur avec turbine de fond. On a agité pendant 3 min à 1500tr/mn. Puis on a ajouté 3,0g de capsules de l'exemple F en homogénéisant le mélange. On a obtenu, après tamisage, une poudre blanc-beige.

Après pulvérisation de la composition sur les cheveux, on constate lorsque le sujet transpire ou au contact du sébum qu'une libération du parfum se produit au cours de la journée.

**Exemple 7 : Préparation d'une poudre libre désodorisante avant la composition suivante** :

| Ingrédients | (% en poids) |
|---|---|
| Talc | 34,4 |
| Amidon de maïs | 30 |
| Perlite | 30 |
| Capsules de parfum F | 5,0 |
| Acide salicylique | 0,1 |
| Octane-1.2-diol | 0,5 |
| Total | 100,00 |

Les capsules de l'exemple F peuvent être remplacées par les capsules des exemples A à E et G, H décrites précédemment.

On a introduit 17,2g de talc, 15,0g d'amidon de maïs, 15,0g de perlite et 0,05g d'acide salicylique dans une cuve équipée d'un agitateur. On a homogénéisé le mélange sous agitation vive (1500tr/mn). Puis on a ajouté 2,5g de capsules de l'exemple F en homogénéisant le mélange avec. On a ajouté enfin 0,25g d'octanediol sous une agitation lente. Après on a tamisé le mélange à l'aide d'un tamis de 250 microns pour obtenir une poudre homogène. On a ainsi obtenu une poudre blanc-beige.

La composition appliquée sur le corps libère le parfum au cours de la journée au contact de la transpiration ou de l'humidité.

**Exemple 8 : Fond de teint poudre compactée**

On a préparé une poudre compactée ayant la composition suivante

Phase A

| | |
|---|---|
| Perlite | 49% |
| Talc | 36,8% |
| Oxydes de fer | 2,4% |
| Oxyde de titane | 3,5% |
| Phenyl trimethicone (Dow Corning 556 de chez Dow Corning) | 3 % |
| Triisostéarate de glycérol | 3 % |
| Conservateur | 0,5 % |

Phase B

Capsules de parfum de l'exemple A      1,8%

Les capsules de l'exemple A peuvent être remplacées par les capsules des exemples B à H décrites précédemment.

Les ingrédients de la phase A ont été mélangés puis broyés, puis les capsules de parfums ont été ajoutées. Le mélange obtenu a été tamisé puis on a versé 9 g du mélange dans une coupelle ronde de 5 cm de diamètre et pressé la poudre sous une pression de 100 bars ($10^7$ Pa)

On a appliqué la poudre compactée sur la peau. Après une minute on a évalué l'intensité de l'odeur du parfum (APPLICATION) en la notant sur une échelle de 0 à 10. 2 heures et 4 heures plus tard on a réévalué à nouveau l'intensité de l'odeur de parfum (AV) avant un ajout d'eau d'environ 0,1 g (trois sprays) sur la composition déposée. On a attendu 30 secondes puis évalué l'intensité de l'odeur de parfum (AP).

| | Intensité odeur T0 | Intensité odeur T2h | | | Intensité odeur T4h | | |
|---|---|---|---|---|---|---|---|
| **Poudre compactée** | **APPLICATION** | **AV** | **AP** | Δ | **AV** | **AP** | Δ |

(suite)

| Poudre compactée | Intensité odeur T0 | Intensité odeur T2h | | | Intensité odeur T4h | | |
|---|---|---|---|---|---|---|---|
| | APPLICATION | AV | AP | Δ | AV | AP | Δ |
| Exemple 8 | 5,0 | 1,5 | 8,5 | 7 | 4,0 | 7,0 | 3,0 |

AV = avant ajout d'eau ; AP = après ajout d'eau ;
Δ= amplitude de différence d'intensité olfactive (AV - AP)
Echelle d'intensité d'odeur de parfum : 0 à 10 (0= inodore; 10= odeur très intense / saturée).

On a ainsi observé à T0 que la poudre compactée présente une odeur de parfum moyenne qui diminue fortement en étant faible à 2h après l'application. On a également observé que la pulvérisation d'eau sur le produit à T2h et T4h conduit à une augmentation de l'intensité de l'odeur de parfum, ce qui montre une libération importante de parfum.

**Revendications**

1.  Composition cosmétique ou dermatologique anhydre sous forme de poudre libre ou compacte comprenant:

    1) au moins des particules à libération d'agent bénéfique comprenant un cœur contenant au moins un agent bénéfique et une enveloppe entourant le coeur ; ladite enveloppe étant constituée d'au moins un polysaccharide modifié hydrophobe choisi parmi les $C_5$-$C_{20}$-alcényl succinates d'amidon et d'au moins un glucide hydrosoluble choisi parmi les maltodextrines ayant un dextrose équivalent allant de 4 à 20 ; lesdites particules présentant simultanément une densité de poudre versée allant de 300,0g/l à 600,0g/l et une densité absolue supérieure à 1,0 ;
    les dites particules étant sphériques et ayant un diamètre moyen en nombre allant de 1 à 30μm, et un diamètre moyen en volume allant de 5 à 150μm ;
    et
    2) éventuellement au moins une charge autre que lesdites particules.

2.  Composition selon la revendication 1, comprenant un milieu physiologiquement acceptable.

3.  Composition selon la revendication 1 ou 2, où les particules sont sphériques et en particulier ont un diamètre moyen en nombre allant de 2 à 15 μm, de préférence allant de 5 à 10 μm et un diamètre moyen en volume allant de 10 à 100 μm , de préférence allant de 20 à 80 μm.

4.  Composition selon l'une quelconque des revendications 1 à 3, où le polysaccharide modifié hydrophobe est l'octényle succinate d'amidon sodique.

5.  Composition selon l'une quelconque des revendications 1 à 4, où le polysaccharide modifié hydrophobe représente de 20 à 90% en poids, notamment 30 à 80% en poids, mieux 40 à 70% en poids, et encore mieux 40 à 60% en poids par rapport au poids total de l'enveloppe de la particule.

6.  Composition selon l'une quelconque des revendication 1 à 5, où le glucide hydrosoluble est choisi les maltodextrines ayant un Dextrose Equivalent allant de 12 à 20.

7.  Composition selon l'une quelconque des revendications précédentes, où le ou les glucides (s) hydrosoluble(s) représentent de 10 à 80% en poids, de préférence de 15 à 70% en poids, plus préférentiellement de 20 à 65% en poids, et encore mieux de 40 à 60% en poids par rapport au poids total de l'enveloppe de la

8.  Composition selon l'une quelconque des revendications précédentes, où l'enveloppe des particules à libération d'agent bénéfique est constituée

    a) d'au moins un $C_5$-$C_{20}$-alcenyl succinate d'amidon dans une quantité allant de 20 à 90% en poids, notamment 30 à 80% en poids, mieux 40 à 70% en poids, et encore mieux 40 à 60% en poids par rapport au poids total de l'enveloppe de la particule et
    b) au moins une maltodextrine ayant un Dextrose Equivalent allant de 4 à 20 et de préférence allant de 12 à 20, dans une quantité allant de 10 à 80% en poids, de préférence de 15 à 70% en poids, plus préférentiellement de 20 à 65% en poids, et encore mieux de 40 à 60% en poids par rapport au poids total de l'enveloppe de la particule.

9. Composition selon l'une quelconque des revendications précédentes, où les particules à libération d'agent bénéfique sont susceptibles d'être obtenues selon un procédé comprenant au moins les étapes suivantes :

- on prépare une solution aqueuse constituée du mélange du glucide hydrosoluble et du polysaccharide modifié hydrophobe puis on ajoute l'agent bénéfique et on agite de façon à former une émulsion ; et
- on homogénéise ladite émulsion ainsi formée sous haute pression à une pression allant de 10 à 200 bars et plus préférentiellement de 20 à 200 bars ; et
- on pulvérise ladite émulsion dans une chambre de séchage, et
- l'eau est extraite pendant une durée de préférence ne dépassant pas 3 heures, et plus préférentiellement ne dépassant pas 30 minutes, avec un fluide sous pression tel que le dioxyde de carbone, de préférence à l'état supercritique de préférence à une pression d'au moins 0,3XPc et à une température d'au moins Tc-60°C avec Pc correspondant à la pression critique du gaz et Tc la température critique du gaz, de façon à obtenir les particules à libération d'agent bénéfique.

10. Composition selon l'une quelconque des revendications précédentes, où les agents bénéfiques sont choisis parmi :

(i) les corps gras ;
(ii) les substances parfumantes ;
(iii) les principes actifs pharmaceutiques ;
(iv) les actifs cosmétiques

11. Composition selon l'une quelconque des revendications précédentes, où les agents bénéfiques sont choisis parmi les substances parfumantes et plus particulièrement celles choisies parmi les notes de cœur et/ou de tête et encore plus particulièrement choisies parmi :

Acétate de benzyle
Acétate de géranyle
Acétate de cis-3-hexenyle
Aldéhyde $C_{18}$ ou nonalactone
Acétate de décyle
Allyl amyl glycolate (citral)
Acétate d'éthyle
Acétate de butyle
Allyl 3-cyclohexylpropionate
Acétate de linalyle
Alcool Phényléthylique
Acétate d'héxyle
Berryflor ou éthyl-6-(acétyloxy)-hexanoate
Acétate d'isoamyle
Caproate d'allyle
Amarocite ou 6,6-diméthoxy-2,5,5-triméthylhex-2-ène
Citral lemarome N ou 3,7-diméthylocta-2,6-diénal
Canthoxal ou anisyl propanal
Claritone ou 2,4,4,7-tétraméthyloct-6-èn-3-one
Ethyl-2méthyl butyrate
Dihydromyrcénol
Cis-3 hexenol
Hédione ou méthyl dihydrojasmonate
L-carvone
Heptanoate d'allyle
Limonène
Néobuténone alpha ou 1-(5,5-diméthyl-1-cyclohexenyl)-pent-4-èn-1-one
Méthylheptènone
Toscanol ou 4-(cyclopropylméthyl)-phénylméthyl éther

myrcenol super ou 2-methyl-6-methylideneoct-7-en-2-ol
Decalactone
Acétate de stéaryle
Oxyde de rose
Linalool
Triplai ou 2,4-dimethylcyclohex-3-ène-1-carbaldéhyde
Mélonal ou 2,6-dimethylhept-5-ènal
1-(1,2,3,4,5,6,7,8-octahydro-2,3,8,8-tetraméethyl-2-naphthyl)éthan-1-one
Hexylcinnamal
Tétrahydro-2-isobutyl-4-méthylpyrann-4-ol
Salicylate d'hexyle
1,4-Dioxacycloheptadécane-5,17-dione
et leurs mélanges

**12.** Composition selon l'une quelconque des revendications précédentes, où les particules comprennent au moins une ou plusieurs substances parfumantes ayant une pression de vapeur saturante à 25°C supérieure ou égale à 10,0 Pa et, préférentiellement, la ou lesdites substances parfumantes représentent de 50 à 100% en poids, plus préférentiellement de 60 à 100% en poids, encore plus préférentiellement de 70 à 100% en poids, et encore mieux de 80 à 100% en poids du poids total des substances parfumantes présentes dans les particules

**13.** Composition selon l'une quelconque des revendications précédentes, **caractérisée par le fait que**

a) les particules comprennent au moins une substance parfumante et
b) la composition comprend en plus au moins une substance parfumante sous forme libre, identique ou différente de ladite substance parfumante présente dans lesdites particules.

**14.** Composition selon l'une quelconque des revendications 1 à 12, **caractérisée par le fait qu'**elle contient exclusivement une ou plusieurs substances parfumantes encapsulées dans les particules.

**15.** Composition selon l'une quelconque des revendications précédentes, comprenant au moins un agent de coloration sous forme libre et/ou encapsulée en particulier choisi parmi les nacres, les pigments, les particules réfléchissantes, et leur(s) mélange(s) et, plus particulièrement dans laquelle les particules comprennent au moins une substance parfumante, et encore plus particulièrement, où la composition comprend en plus au moins une substance parfumante sous forme libre, identique ou différente de ladite substance parfumante présente dans lesdites particules.

**16.** Composition selon l'une quelconque des revendications précédentes, comprenant au moins un actif déodorant et/au au moins un actif anti-transpirant sous forme libre et/ou sous forme encapsulée et, plus particulièrement, dans laquelle les particules à libération d'agent bénéfique comprennent au moins une substance parfumante, et encore plus particulièrement, où la composition comprend en plus au moins une substance parfumante sous forme libre, identique ou différente de ladite substance parfumante présente dans lesdites particules.

**17.** Composition selon l'une quelconque des revendications précédentes, **caractérisée par le fait qu'**elle comprend des charges en une teneur allant de 20 à 99,9 % en poids, de préférence de 40 à 99,8% en poids, plus préférentiellement encore de 60 à 99% en poids, par rapport au poids total de la composition.

**18.** Procédé cosmétique de soin et/ou d'hygiène et/ou de parfumage et/ou de de conditionnement et/ou de maquillage d'une matière kératinique humaine consistant à appliquer sur ladite matière kératinique humaine une composition selon l'une quelconque des revendications précédentes.

**19.** Procédé cosmétique de traitement des odeurs corporelles et éventuellement de la transpiration humaine consistant à appliquer sur une matière kératinique une composition selon la revendication 16.

**20.** Produit de consommation, **caractérisé par le fait qu'**il est constituée par une composition telle que définie selon l'une quelconque des revendications 1 à 17.

**Patentansprüche**

1. Wasserfreie kosmetische oder dermatologische Zusammensetzung in Form eines lockeren oder verdichteten Pulvers, Folgendes umfassend:

   1) zumindest Partikel, aus denen ein Mittel mit Nutzwirkung freigesetzt wird, wobei sie einen Kern umfassen, welcher mindestens ein Mittel mit Nutzwirkung enthält, sowie eine Hülle, welche den Kern umgibt; wobei die Hülle aus mindestens einem hydrophob modifizierten Polysaccharid, welches aus den $C_5$-$C_{20}$-Alkenylsuccinaten von Stärke ausgewählt ist, und aus mindestens einem wasserlöslichen Kohlenhydrat besteht, welches aus den Maltodextrinen mit einem Dextrose-Äquivalent im Bereich von 4 bis 20 ausgewählt ist; wobei die Partikel sowohl eine Pulverschüttdichte im Bereich von 300,0 g/L bis 600,0 g/L als auch eine Absolutdichte von mehr als 1,0 aufweisen;
   wobei die Partikel kugelförmig sind und einen zahlenbezogenen mittleren Durchmesser im Bereich von 1 bis 30 $\mu$m und einen volumenbezogenen mittleren Durchmesser im Bereich des von 5 bis 150 $\mu$m aufweisen; und
   2) möglicherweise mindestens einen Füllstoff, der nicht den Partikeln entspricht.

2. Zusammensetzung nach Anspruch 1, die ein physiologisch unbedenkliches Medium umfasst.

3. Zusammensetzung nach Anspruch 1 oder 2, wobei die Partikel kugelförmig sind und insbesondere einen zahlenbezogenen mittleren Durchmesser im Bereich von 2 bis 15 um, vorzugsweise im Bereich von 5 bis 10 $\mu$m, und einen volumenbezogenen Durchmesser im Bereich von 10 bis 100 $\mu$m, vorzugsweise im Bereich von 20 bis 80 $\mu$m, haben.

4. Zusammensetzung nach einem beliebigen der Ansprüche 1 bis 3, wobei es sich bei dem hydrophob modifizierten Polysaccharid um Stärkenatriumoctenylsuccinat handelt.

5. Zusammensetzung nach einem beliebigen der Ansprüche 1 bis 4, wobei das hydrophob modifizierte Polysaccharid 20 bis 90 Gewichts-%, insbesondere 30 bis 80 Gewichts-%, besser 40 bis 70 Gewichts-%, und besser noch 40 bis 60 Gewichts-% ausmacht, bezogen auf das Gesamtgewicht der Hülle des Partikels.

6. Zusammensetzung nach einem beliebigen der Ansprüche 1 bis 5, wobei das wasserlösliche Kohlenhydrat aus den Maltodextrinen mit einem Dextrose-Äquivalent im Bereich von 12 bis 20 ausgewählt ist.

7. Zusammensetzung nach einem beliebigen der vorhergehenden Ansprüche, wobei das oder die wasserlösliche(n) Kohlenhydrate 10 bis 80 Gewichts-%, vorzugsweise 15 bis 70 Gewichts-%, stärker bevorzugt 20 bis 65 Gewichts-%, und besser noch 40 bis 60 Gewichts-% ausmachen, bezogen auf das Gesamtgewicht des Partikels.

8. Zusammensetzung nach einem beliebigen der vorhergehenden Ansprüche, wobei die Hülle der Partikel, aus welchen ein Mittel mit Nutzwirkung freigesetzt wird, aus Folgendem besteht

   a) mindestens einem $C_5$-$C_{20}$-Alkenylsuccinat von Stärke in einer Menge im Bereich von 20 bis 90 Gewichts-%, insbesondere 30 bis 80 Gewichts-%, besser 40 bis 70 Gewichts-%, und noch besser 40 bis 60 Gewichts-%, bezogen auf das Gesamtgewicht der Hülle des Partikels, und
   b) mindestens ein Maltodextrin mit einem Dextrose-Äquivalent im Bereich von 4 bis 20 und vorzugsweise im Bereich von 12 bis 20, in einer Menge im Bereich von 10 bis 80 Gewichts-%, vorzugsweise von 15 bis 70 Gewichts-%, stärker bevorzugt von 20 bis 65 Gewichts-%, und noch besser von 40 bis 60 Gewichts-%, bezogen auf das Gesamtgewicht der Hülle des Partikels.

9. Zusammensetzung nach einem beliebigen der vorhergehenden Ansprüche, wobei die Partikel, aus welchen ein Mittel mit Nutzwirkung freigesetzt wird, gemäß einem Verfahren erhalten werden können, das mindestens die folgenden Schritte umfasst:

   - Herstellen einer wässrigen Lösung, die aus der Mischung des wasserlöslichen Kohlenhydrats mit dem hydrophob modifizierten Polysaccharid besteht, woraufhin das Mittel mit Nutzwirkung zugesetzt und derart gerührt wird, dass sich eine Emulsion bildet; und
   - Homogenisieren der auf diese Weise gebildeten Emulsion unter hohem Druck, bei einem Druck im Bereich von 10 bis 200 bar und stärker bevorzugt von 20 bis 200 bar; und

- versprühen der Emulsion in einer Trockenkammer, und
- Entfernen von Wasser während einer Dauer, die vorzugsweise höchstens 3 Stunden beträgt, wobei sie stärker bevorzugt höchstens 30 Minuten beträgt, mit einem unter Druck stehenden Fluid wie etwa Kohlendioxid, vorzugsweise im überkritischen Zustand, vorzugsweise bei einem Druck von mindestens 0,3 x Pc und einer Temperatur von mindestens Tc - 60 °C, wobei Pc dem kritischen Druck des Gases entspricht und Tc der kritischen Temperatur des Gases entspricht, sodass die Partikel erhalten werden, aus welchen ein Mittel mit Nutzwirkung freigesetat wird.

10. Zusammensetzung nach einem beliebigen der vorhergehenden Ansprüche, wobei die Mittel mit Nutzwirkung aus den folgenden ausgewählt sind:

(i) Fettstoffen;
(ii) Duftstoffen;
(iii) pharmazeutischen Wirkstoffen;
(iv) kosmetischen Wirkstoffen.

11. Zusammensetzung nach einem beliebigen der vorhergehenden Ansprüche, wobei die Mittel mit Nutzwirkung aus den Duftstoffen ausgewählt sind, und insbesondere aus denjenigen, welche aus den Herz- und/oder Kopfnoten ausgewählt sind, wobei sie insbesondere aus den folgenden ausgewählt sind:

Benzylactetat
Geranylacetat
cis-3-Hexenylacetat
$C_{18}$-Aldehyd oder Nonalacton
Decylacetat
Allylamylglycolat (Citral)
Ethylacetat
Butylactetat
Allyl-3-cyclohexylpropionat
Linalylacetat
Phenylethylalkohol
Hexylacatat
Berryflor oder Ethyl-6-(acetyloxy)-hexanoat Isoamylacetat
Allylcaproat
Amarocite oder 6,6-Dimethoxy-2,5,5-trimethylhex-2-eh
Citral lemarome N oder 3,7-Dimethylocta-2,6-dienal Canthoxal oder Anisylpropanal
Claritone oder 2,4,4,7-Tetramethyloct-6-en-3-on Ethyl-2-methylbutyrat
Dihydromyrcenol
cis-3-Hexenol
Hedion oder Methyldihydrojasmonat
L-Carvon
Allylheptanoat
Limonen
Neobutenon alpha oder 1-(5,5-Dimethyl-1-cyclohexenyl)-pent-4-en-1-on
Methylheptenon
Toscanol oder 4-(Cyclopropylmethyl)-phenylmethylether
Myrcenol super oder 2-Methyl-6-methylidenoct-7-en-2-ol
Decalacton
Stearylacetat
Rosenoxid
Linalool
Triplal oder 2,4-Dimethylcyclohex-3-en-1-carbaldehyd
Melonal oder 2,6-Dimethylhept-5-enal
1-(1,2,3,4,5,6,7,8-Octahydro-2,3,8,8-tetramethyl-2-naphthyl)-ethan-1-on
Hexylcinnamal
Tetrahydro-2-isobutyl-4-methylpyran-4-ol
Hexylsalicylat
1,4-Dioxacycloheptadecan-5,17-dion

und deren Mischungen.

12. Zusammensetzung nach einem beliebigen der vorhergehenden Ansprüche, wobei die Partikel mindestens einen oder mehrere Duftstoffe umfassen, welche bei 25 °C einen Sättigungsdampfdruck von mindestens 10,0 Pa haben, wobei dieser oder diese Duftstoffe vorzugweise 50 bis 100 Gewichts-%, stärker bevorzugt 60 bis 100 Gewichts-%, noch stärker bevorzugt 70 bis 100 Gewichts-%, und noch besser 80 bis 100 Gewichts-% des Gesamtgewichts der Duftstoffe ausmachen, welche in den Partikeln vorliegen.

13. Zusammensetzung nach einem beliebigen der vorhergehenden Ansprüche, **dadurch gekennzeichnet, dass**

    a) die Partikel mindestens einen Duftstoff umfassen und
    b) die Zusammensetzung darüber hinaus mindestens einen Duftstoff in freier Form umfasst, wobei dieser gleichartig oder verschiedenartig mit/von dem Duftstoff ist, welcher in den Partikeln vorliegt.

14. Zusammensetzung nach einem beliebigen der Ansprüche 1 bis 12, **dadurch gekennzeichnet, dass** sie ausschließlich einen oder mehrere Duftstoffe enthält, die in den Partikeln verkapselt sind.

15. Zusammensetzung nach einem beliebigen der vorhergehenden Ansprüche, wobei sie mindestens einen Farbstoff in freier und/oder verkapselter Form umfasst, der insbesondere aus den Perlglanzstoffen, den Pigmenten, den reflektierenden Partikeln und deren Mischung(en) ausgewählt ist, wobei die darin vorliegenden Partikel insbesondere mindestens einen Duftstoff umfassen und wobei die Zusammensetzung darüber hinaus ganz besonders mindestens einen Duftstoff in freier Form umfasst, der gleichartig oder verschiedenartig mit/von dem Duftstoff ist, welcher in den Partikeln vorliegt.

16. Zusammensetzung nach einem beliebigen der vorhergehenden Ansprüche, wobei sie mindestens einen desodorierenden Wirkstoff und/oder mindestens einen Antitranspiranten in freier und/oder verkapselter Form umfasst, und wobei die darin vorliegenden Partikel, aus welchen ein Mittel mit Nutzwirkung freigesetzt wird, insbesondere mindestens einen Duftstoff umfassen und wobei die Zusammensetzung darüber hinaus ganz besonders mindestens einen Duftstoff in freier Form umfasst, der gleichartig oder verschiedenartig mit/von dem Duftstoff ist, welcher in den Partikeln vorliegt.

17. Zusammensetzung nach einem beliebigen der vorhergehenden Ansprüche, **dadurch gekennzeichnet, dass** sie Füllstoffe zu einem Gehalt im Bereich von 20 bis 99,9 Gewichts-%, vorzugsweise von 40 bis 99,8 Gewichts-%, stärker bevorzugt von 60 bis 99 Gewichts-%, umfasst, bezogen auf das Gesamtgewicht der Zusammensetzung.

18. Kosmetisches Verfahren zum Pflegen und/oder zur Hygienewahrung und/oder zum Parfümieren und/oder zum Spülen und/oder zum Ändern des Erscheinungsbildes eines menschlichen Keratinstoffs, wobei es darin besteht, eine Zusammensetzung nach einem beliebigen der vorhergehenden Ansprüche auf den menschlichen Keratinstoff aufzubringen.

19. Kosmetisches Verfahren zum Behandeln von Körpergeruch sowie möglicherweise der Schweißbildung beim Menschen, wobei es darin besteht, eine Zusammensetzung nach Anspruch 16 auf einen Keratinstoff aufzubringen.

20. Verbrauchsprodukt, **dadurch gekennzeichnet, dass** es aus einer Zusammensetzung besteht, wie sie nach einem beliebigen der Ansprüche 1 bis 17 definiert ist.

**Claims**

1. Anhydrous cosmetic or dermatological composition in the form of a loose or compact powder, comprising:

    1) at least particles with release of a beneficial agent comprising a core containing at least one beneficial agent and an envelope surrounding the core; said envelope being constituted of at least one hydrophobically modified polysaccharide chosen from starch ($C_5$-$C_{20}$) alkenyl succinates and of at least one water-soluble carbohydrate chosen from maltodextrins having a dextrose equivalent ranging from 4 to 20; said particles simultaneously having a poured powder density ranging from 300.0 g/l to 600.0 g/l and an absolute density of greater than 1.0; said particles being spherical and having a number-mean diameter ranging from 1 to 30 $\mu$m, and a volume-mean diameter ranging from 5 to 150 $\mu$m;

37

and

2) optionally at least one filler other than said particles.

2. Composition according to Claim 1, comprising a physiologically acceptable medium.

3. Composition according to Claim 1 or 2, in which the particles are spherical and in particular have a number-mean diameter ranging from 2 to 15 $\mu$m, preferably ranging from 5 to 10 $\mu$m and a volume-mean diameter ranging from 10 to 100 $\mu$m, preferably ranging from 20 to 80 $\mu$m.

4. Composition according to any one of Claims 1 to 3, in which the hydrophobically modified polysaccharide is sodium starch octenyl succinate.

5. Composition according to any one of Claims 1 to 4, in which the hydrophobically modified polysaccharide represents from 20% to 90% by weight, in particular 30% to 80% by weight, better still 40% to 70% by weight, and even better still 40% to 60% by weight, relative to the total weight of the envelope of the particle.

6. Composition according to any one of Claims 1 to 5, in which the water-soluble carbohydrate is chosen from maltodextrins having a dextrose equivalent ranging from 12 to 20.

7. Composition according to any one of the preceding claims, in which the water-soluble carbohydrate(s) represent from 10% to 80% by weight, preferably from 15% to 70% by weight, more preferentially from 20% to 65% by weight, and even better still from 40% to 60% by weight, relative to the total weight of the envelope of the particle.

8. Composition according to any one of the preceding claims, in which the envelope of the particles with release of a beneficial agent is constituted of

a) at least one starch ($C_5$-$C_{20}$) alkenyl succinate in an amount ranging from 20% to 90% by weight, in particular 30% to 80% by weight, better still 40% to 70% by weight, and even better still 40% to 60% by weight, relative to the total weight of the envelope of the particle, and

b) at least one maltodextrin having a dextrose equivalent ranging from 4 to 20 and preferably ranging from 12 to 20, in an amount ranging from 10% to 80% by weight, preferably from 15% to 70% by weight, more preferentially from 20% to 65% by weight, and even better still from 40% to 60% by weight, relative to the total weight of the envelope of the particle.

9. Composition according to any one of the preceding claims, in which the particles with release of a beneficial agent are able to be obtained according to a process comprising at least the following steps:

- an aqueous solution constituted of the mixture of the water-soluble carbohydrate and of the hydrophobically modified polysaccharide is prepared, then the beneficial agent is added and stirring is carried out so as to form an emulsion; and
- said emulsion thus formed is homogenized under high pressure at a pressure ranging from 10 to 200 bar and more preferentially from 20 to 200 bar; and
- said emulsion is sprayed in a drying chamber, and
- the water is extracted for a period of time preferably not exceeding 3 hours, and more preferentially not exceeding 30 minutes, with a fluid under pressure such as carbon dioxide, preferably in the supercritical state, preferably at a pressure of at least 0.3XPc and at a temperature of at least Tc-60°C with Pc corresponding to the critical pressure of the gas and Tc corresponding to the critical temperature of the gas so as to obtain the particles with release of a beneficial agent.

10. Composition according to any one of the preceding claims, in which the beneficial agents are chosen from:

(i) fatty substances;
(ii) fragrancing substances;
(iii) pharmaceutical active ingredients;
(iv) cosmetic active agents.

11. Composition according to any one of the preceding claims, in which the beneficial agents are chosen from fragrancing substances and more particularly those chosen from heart notes and/or top notes and even more particularly chosen from:

Benzyl acetate
Geranyl acetate
Cis-3-hexenyl acetate
$C_{18}$ aldehyde or nonalactone
Decyl acetate
Allyl amyl glycolate (citral)
Ethyl acetate
Butyl acetate
Allyl 3-cyclohexylpropionate
Linalyl acetate
Phenylethyl alcohol
Hexyl acetate
Berryflor or ethyl 6-(acetyloxy)hexanoate isoamyl acetate
Allyl caproate
Amarocite or 6,6-dimethoxy-2,5,5-trimethylhex-2-ene
Citral lemarome N or 3,7-dimethylocta-2,6-dienal
Canthoxal or anisyl propanol
Claritone or 2,4,4,7-tetrainethyloct-6-en-3-one
Ethyl 2-methylbutyrate
Dihydromyrcenol
Cis-3-hexenol
Hedione or methyl dihydrojasmonate
L-carvone
Allyl heptanoate
Limonene
Neobutenone alpha or 1-(5,5-dimethyl-1-cyclohexenyl)pent-4-en-1-one
Methylheptenone
Toscanol or 4-(cyclopropylmethyl)phenyl methyl ether
Myrcenol Super or 2-methyl-6-methylideneoct-7-en-2-ol
Decalactone
Stearyl acetate
Rose oxide
Linalool
Triplal or 2,4-dimethylcyclohex-3-ene-1-carbaldehyde
Melonal or 2,6-dimethylhept-5-enal
1-(1,2,3,4,5,6,7,8-octahydro-2,3,8,8-tetramethyl-2-naphthyl)ethan-1-one
Hexylcinnamal
Tetrahydro-2-isobutyl-4-methylpyran-4-ol
Hexyl salicylate
1,4-Dioxacycloheptadecane-5,17-dione
and mixtures thereof.

12. Composition according to any one of the preceding claims, in which the particles comprise at least one or more fragrancing substances having a saturation vapour pressure at 25°C of greater than or equal to 10.0 Pa and, preferentially, said fragrancing substahce(s) represent from 50% to 100% by weight, more preferentially from 60% to 100% by weight, even more preferentially from 70% to 100% by weight, and even better still from 80% to 100% by weight of the total weight of the fragrancing substances present in the particles.

13. Composition according to any one of the preceding claims, **characterized in that**

a) the particles comprise at least one fragrancing substance and
b) the composition also comprises at least one fragrancing substance in free form, which may be identical to or different from said fragrancing substance present in said particles.

14. Composition according to any one of Claims 1 to 12, **characterized in that** it exclusively contains one or more fragrancing substances encapsulated in the particles.

15. Composition according to any one of the preceding claims, comprising at least one colouring agent in free and/or encapsulated form, in particular chosen from nacreous, pigments and reflective particles, and mixture (s) thereof, and more particularly in which the particles comprise at least one fragrancing substance, and even more preferentially in which the composition also comprises at least one fragrancing substance in free form, which may be identical to or different from said fragrancing substance present in said particles.

16. Composition according to any one of the preceding claims, comprising at least one deodorant active agent and/or at least one antiperspirant active agent in free form and/or in encapsulated form, and more particularly in which the particles with release of a beneficial agent comprise at least one fragrancing substance, and even more particularly in which the composition also comprises at least one fragrancing substance in free form, which may be identical to or different from said fragrancing substance present in said particles.

17. Composition according to any one of the preceding claims, **characterized in that** it comprises fillers in a content ranging from 20% to 99.9% by weight, preferably from 40% to 99.8% by weight, even more preferentially from 60% to 99% by weight, relative to the total weight of the composition.

18. Cosmetic process for caring for and/or for the hygiene of and/or for fragrancing and/or for conditioning and/or for making up a human keratin material, consisting in applying to said human keratin material a composition according to any one of the preceding claims.

19. Cosmetic process for treating body odour and optionally human perspiration, consisting in applying to a keratin material a composition according to Claim 16.

20. Consumer product, **characterized in that** it is constituted of a composition as defined according to any one of Claims 1 to 17.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5508259 A
- DE 102008035013
- US 20040029750 A
- US 6200949 B
- EP 1917098 B1
- JP 09188830 A
- JP 10158450 A
- JP 10158541 A

- JP 07258460 A
- JP 05017710 A
- US 5002698 A
- US 2005063928 A
- US 2005084464 A
- US 2005084474 A
- EP 1658863 A
- US 6916465 B

**Littérature non-brevet citée dans la description**

- International Cosmetic Ingredient Dictionnary and Handbook. The Cosmetic, Toiletry, and Fragrance Association, 1997, 371-386, 524-528
- **S. ARCTANDER.** *Perfume and Flavor Chemicals,* 1969

- **S. ARCTANDER.** *Perfume and Flavor Materials of Natural Origin,* 1960
- Flavor and Fragrance Materials. Allured Publishing Co, 1991